# EUROPÄISCHE PATENTSCHRIFT

(11) **EP 4 473 051 B1**
(45) Veröffentlichungstag und Bekanntmachung des Hinweises auf die Patenterteilung: **12.11.2025**
(21) Anmeldenummer: 23703688.4
(22) Anmeldetag: 20.01.2023
(51) Int. Cl.: C08K 5/3437, B60C 1/00, C08K 3/04

(54) **VERBINDUNG, KAUTSCHUKMISCHUNG ENTHALTEND DIE VERBINDUNG, FAHRZEUGREIFEN, DER DIE KAUTSCHUKMISCHUNG IN WENIGSTENS EINEM BAUTEIL AUFWEIST, VERFAHREN ZUR HERSTELLUNG DER VERBINDUNG SOWIE VERWENDUNG DER VERBINDUNG ALS ALTERUNGSSCHUTZMITTEL UND/ODER ANTIOXIDATIONSMITTEL**
COMPOUND, RUBBER BLEND CONTAINING THE COMPOUND, VEHICLE TIRE COMPRISING THE RUBBER BLEND IN AT LEAST ONE COMPONENT, PROCESS FOR PREPARING THE COMPOUND, AND USE OF THE COMPOUND AS AN ANTI-AGING AGENT AND/OR ANTIOXIDANT
COMPOSÉ, MÉLANGE DE CAOUTCHOUC CONTENANT LE COMPOSÉ, PNEU DE VÉHICULE COMPRENANT LE MÉLANGE DE CAOUTCHOUC DANS AU MOINS UN COMPOSANT, PROCÉDÉ DE PRÉPARATION DU COMPOSÉ, ET UTILISATION DU COMPOSÉ EN TANT QU'AGENT ANTI-VIEILLISSEMENT ET/OU ANTIOXYDANT

(30) Priorität: 31.01.2022 DE 102022200974
(43) Veröffentlichungstag der Anmeldung: 11.12.2024
(73) Patentinhaber: Continental Reifen Deutschland GmbH, 30175 Hannover (DE)
(72) Erfinder: JACOB, Andreas, 30175 Hannover (DE); DAUER, David-Raphael, 30175 Hannover (DE); STROHMEIER, Julian, 30175 Hannover (DE)
(74) Vertreter: Continental Corporation
(86) Internationale Anmeldenummer: PCT/DE2023/200017
(87) Internationale Veröffentlichungsnummer: WO 2023/143677

(56) Entgegenhaltungen:
- EP-A1- 3 517 570
- EP-A2- 2 346 946
- EP-B1- 2 346 946
- DE-A1- 1 694 318
- ZHANG DEKUN ET AL: "Remote Enantioselective Desymmetrization of 9,9-Disubstituted 9,10-Dihydroacridines through Asymmetric Aromatic Aminations", ACS CATALYSIS, vol. 12, no. 23, 15 November 2022 (2022-11-15), US, pages 14609 - 14618, XP093016197, ISSN: 2155-5435, Retrieved from the Internet <URL:https://pubs.acs.org/doi/pdf/10.1021/acscatal.2c04975> DOI: 10.1021/acscatal.2c04975
- UNDERHILL JACK ET AL: "Dioxygen Splitting by a Tantalum(V) Complex Ligated by a Rigid, Redox Non-Innocent Pincer Ligand**", CHEMISTRY - A EUROPEAN JOURNAL, 29 November 2022 (2022-11-29), DE, XP093016191, ISSN: 0947-6539, Retrieved from the Internet <URL:https://onlinelibrary.wiley.com/doi/full-xml/10.1002/chem.202203266> DOI: 10.1002/chem.202203266
- KISELYOV ALEXANDER: "A Domino Cyclization Reaction of Iminium Salts: A Convenient Route to Hexahydrobenzo[ b ]phenanthrolines", SYNLETT, vol. 2006, no. 03, 1 February 2006 (2006-02-01), DE, pages 0391 - 0394, XP093017242, ISSN: 0936-5214, DOI: 10.1055/s-2006-926236

## Beschreibung

Die Erfindung betrifft eine Verbindung, eine Kautschukmischung enthaltend die Verbindung, einen Fahrzeugreifen, der die Kautschukmischung in wenigstens einem Bauteil aufweist, Verfahren zur Herstellung der Verbindung sowie die Verwendung der Verbindung als Alterungsschutzmittel und/oder Antioxidationsmittel.

Es ist bekannt, dass in Fahrzeugreifen und technischen Gummiartikeln polymere Materialien, wie insbesondere Kautschuke, eingesetzt werden.

Naturkautschuk, synthetische Polymere (wie IR, BR, SSBR, ESBR, etc.) aber auch natürliche sowie synthetische Öle, Fette und Schmiermittel unterliegen bei längerer Lagerung und vor allem in der Zielanwendung, die oft bei höheren Temperaturen abläuft, Oxidationsreaktionen, die sich nachteilig auf die ursprünglichen gewünschten Eigenschaften auswirken. Je nach Art des Polymers werden die Polymerketten, bis hin zu einer Verflüssigung des Materials, verkürzt oder es kommt zur nachträglichen Härtung des Werkstoffes.

Alterungsschutzmittel tragen daher maßgeblich zur Langlebigkeit von Fahrzeugreifen und anderen technischen Gummiartikeln bei. Bekannte Alterungsschutzmittel sind aromatische Amine, wie beispielsweise 6PPD (N-(1,3-Dimethylbutyl)-N'-phenyl-p-phenylendiamin), IPPD (N-Isopropyl-N'-phenyl-p-phenylendiamin) oder SPPD (N-(1-phenylethyl)-N'-phenyl-p-phenylendiamin).

Diese Moleküle können mit Sauerstoff oder Ozon oder gebildeten Radikalen, wie Alkyl-, Alkoxy- und Alkylperoxyradikalen, reagieren und diese somit abfangen und somit die Polymere vor weiteren Oxidationsreaktionen schützen.

Nachteilig an dieser Substanzklasse ist allerdings der Verdacht, dass diese krebserregend sein könnten.

Alterungsschutzmittel, die insbesondere mit Ozon reagieren und dieses abfangen, werden auch als "Ozonschutzmittel" oder "Antiozonant" bezeichnet.

Eine weitere Problematik im Zusammenhang mit Alterungsschutzmitteln ist die des unerwünschten Ausblühens (engl. "blooming"). Hierbei diffundieren Moleküle des Alterungsschutzmittels aufgrund ihrer nicht so guten Löslichkeit in der umgebenden Polymer-Matrix des Gummiartikels an die Oberfläche des zu schützenden Artikels und bilden dort einen farblich vom restlichen Artikel meist unterscheidbaren Film. Bei Fahrzeugreifen äußert sich dies meist in einer Braunfärbung der ansonsten schwarzen Seitenwand. Neben den ästhetischen Nachteilen sind hiermit Nachteile hinsichtlich der Alterungsschutzwirkung verbunden. Meist werden ausgeblühte Substanzen nämlich entfernt. Hierbei wird zum einen die Gesamtmenge an Alterungsschutzmittel herabgesetzt und zum anderen bewirkt, dass weitere Moleküle des Alterungsschutzmittels nachdiffundieren, sodass die Polymere immer weniger geschützt sind.

Der Erfindung liegt die Aufgabe zugrunde, eine neuartige Verbindung bereitzustellen, die insbesondere als Alterungsschutzmittel in Fahrzeugreifen oder anderen technischen Gummiartikeln verwendet werden kann, und zwar mit einem geringeren Gefahrenpotential bei hinreichender Löslichkeit in der jeweiligen Matrix, beispielsweise und insbesondere im Polymer. Hierdurch soll unter Verringerung der Gesundheitsschädlichkeit ein weiterhin optimaler Schutz vor Sauerstoff und Ozon gewährleistet werden sowie die Tendenz zum Ausblühen (engl. "Blooming") verhindert werden.

Gleichzeitig soll die Verbindung auf besonders energie- und kostensparende Weise herstellbar sein.

Gelöst wird die Aufgabe durch die erfindungsgemäße Verbindung gemäß Anspruch 1, die erfindungsgemäße Kautschukmischung enthaltend die Verbindung sowie den erfindungsgemäßen Fahrzeugreifen, der die erfindungsgemäße Kautschukmischung in wenigstens einem Bauteil aufweist.

Ferner wird die Aufgabe durch das erfindungsgemäße Verfahren zur Herstellung der erfindungsgemäßen Verbindung gelöst.

Die Verbindung gemäß Anspruch 1 weist die allgemeine Formel I) auf: 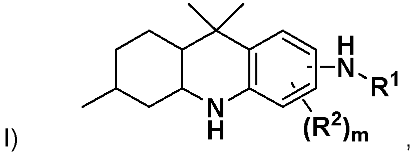
wobei R¹ ausgewählt ist aus der Gruppe bestehend aus xi) aromatischen Resten, wobei die aromatischen Reste optional Substituenten, die ausgewählt sind aus der Gruppe bestehend aus Halogen-Resten, Cyano-Resten, Ester-Resten, Keton-Resten, Ether-Resten und Thioether-Resten, tragen,
und xii) linearen, verzweigten und cyclischen aliphatischen C₃- bis C₁₂-Resten, wobei R¹ optional ein divalenter Rest ist, der mit einer Valenz an den Benzolring angebunden ist; und
   wobei R² ausgewählt ist aus der Gruppe bestehend aus linearen, verzweigten und cyclischen aliphatischen C₁- bis C₁₂-Resten, welche optional einen oder mehrere Halogen-Substituenten tragen, Aryl-Resten, welche optional einen oder mehrere Halogen-Substituenten tragen, und Halogen-Resten, wobei Fluor, Brom und Chlor bevorzugt sind, Cyano-Resten, Ester-Resten, Keton-Resten, Ether-Resten und Thioether-Resten; und wobei m den Wert 0 oder 1 oder 2 oder 3 annimmt.

Dem Fachmann ist klar, dass im Fall von m gleich 0 (null) oder 1 oder 2 anstelle des R² jeweils ein Wasserstoffatom an das entsprechende Kohlenstoffatom des Benzolringes gebunden ist.

Dem Fachmann ist ebenfalls klar, dass die Darstellung der Verknüpfungen von (R²)ₘ sowie R¹HN in den Benzolring des Gerüsts bedeutet, dass diese Gruppen jeweils an jeder Stelle am jeweiligen Benzolring angeordnet sein können, außer natürlich nicht jeweils zwei oder mehrere gleichzeitig an derselben Position, was schon aufgrund der Vierbindigkeit des Kohlenstoffatoms des Benzolrings ausgeschlossen wäre.

Bezeichnungen derart wie "C₃- bis C₁₂-Reste" bedeutet im Rahmen der vorliegenden Erfindung, dass Reste mit 3 bis 12 Kohlenstoffatomen gemeint sind. Unabhängig davon wird "C₁" als Bezeichnung der Position des höchstoxidierten Kohlenstoffatoms bzw. der höchsten Priorität nach der Cahn-Ingold-Prelog-Konvention (CIP) verwendet. Dem Fachmann ist im jeweiligen Kontext klar, was gemeint ist.

Die erfindungsgemäße Verbindung ist ein Octahydroacridin-Derivat und weist gegenüber bekannten Alterungsschutzmitteln auf Basis von Anilin (mögliches Spaltprodukt von 6PPD) ein geringeres Gefahrenpotential auf.

Die erfindungsgemäße Verbindung weist gegenüber dem bekannten Alterungsschutzmittel 6PPD eine vergleichbare Reaktivität gegenüber Sauerstoff, Ozon oder Radikalen auf, wodurch mit der Verbindung gemäß Formel I) eine vergleichbare Schutzwirkung, insbesondere in Fahrzeugreifen und anderen technischen Gummiartikeln, aber auch in Ölen und Schmierstoffen, erzielt wird. Die Erfindung soll aber nicht an einen bestimmten Wirkmechanismus oder eine bestimmte Erklärung gebunden sein.

Die erfindungsgemäße Verbindung ist somit als Ersatz von 6PPD geeignet, dessen Abbauprodukte extrem toxisch für den Silberlachs und damit wohl auch für andere aquatische Organismen sind.

Die erfindungsgemäße Verbindung weist zudem eine sehr gute Löslichkeit in Kautschukmischungen, insbesondere für Fahrzeugreifen und andere technische Gummiartikel, auf. Hierdurch wird ein Ausblühen dieser Verbindung, wie es von vielen Alterungsschutzmitteln bekannt ist, vermieden, was wiederum eine vorteilhafte bzw. verbesserte Alterungsschutzwirkung bedeutet. Je weniger an Alterungsschutzmittel ausblüht, desto weniger Alterungsschutzmittel wird beabsichtigt oder unbeabsichtigt von der Oberfläche des zu schützenden Artikels entfernt und desto weniger Alterungsschutzmittel diffundiert wiederum an die Oberfläche.

Zudem lässt sich die erfindungsgemäße Verbindung mit dem erfindungsgemäßen Verfahren auf vergleichsweise einfache, energie- und kostensparende Weise herstellen. Insbesondere sind bei der Herstellung keine Edelmetallkatalysatoren notwendig. Ferner kann das Verfahren bei Raumtemperatur durchgeführt werden. Zudem kann die ionische Flüssigkeit, welche sowohl als Lösungsmittel als auch als Katalysator dient, nach Aufreinigung durch Extraktion der erfindungsgemäßen Verbindung wiederverwendet werden.

Die erfindungsgemäße Verbindung gemäß Formel I) ist besonders als Alterungsschutzmittel und/oder Ozonschutzmittel in Fahrzeugreifen und/oder anderen technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen geeignet.

Die erfindungsgemäße Verbindung gemäß Formel I) ist besonders zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon geeignet.

Zu Verwendung der Verbindung gemäß Formel I) in den genannten Artikeln oder Stoffen wird diese in einer Zusammensetzung verwendet und in dieser eingemischt verwendet.

Bei Fahrzeugreifen oder anderen technischen Gummiartikeln ist dies insbesondere eine Kautschukmischung.

Ein weiterer Gegenstand der Erfindung ist die Verwendung der erfindungsgemäßen Verbindung gemäß Formel I) in Ölen, Schmierstoffen, wie insbesondere Treib- oder Betriebsstoffen für Motoren. Insbesondere kann die erfindungsgemäße Verbindung somit in Motoren verwendet werden.

Von der Erfindung sind sämtliche vorteilhafte Ausgestaltungen, die sich unter anderem in den Patentansprüchen widerspiegeln, umfasst. Insbesondere sind von der Erfindung auch Ausgestaltungen umfasst, die sich durch Kombination unterschiedlicher Merkmale unterschiedlicher Abstufungen bei der Bevorzugung dieser Merkmale ergeben, sodass auch eine Kombination eines ersten als "bevorzugt" bezeichneten Merkmals oder im Rahmen einer vorteilhaften Ausführungsform beschriebenen Merkmals mit einem weiteren als z. B. "besonders bevorzugt" bezeichneten Merkmal von der Erfindung umfasst ist.

Bevorzugt ist m in Formel I) gleich null.

Bevorzugt weist die erfindungsgemäße Verbindung die Struktur gemäß Formel II) auf: 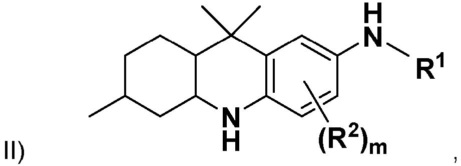 wobei R¹, R² und m wie obenstehend definiert sind.

Gemäß dieser bevorzugten Struktur sind die beiden Stickstoffatome somit in para-Stellung zueinander angeordnet.

Besonders bevorzugt ist m hierbei gleich 0 (null), sodass die Verbindung damit die Struktur gemäß Formel IIa) aufweist: 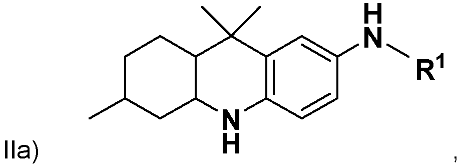 wobei R¹ wie obenstehend definiert ist.

Die Verbindung gemäß den Formeln II) bzw. IIa) lassen sich besonders einfach sowie energie- und kostensparend herstellen und zeigen im Vergleich zu 6PPD eine vergleichbare Reaktivität gegenüber Sauerstoff, Ozon oder Radikalen und damit eine vergleichbare Alterungsschutzwirkung.

Der Rest R¹ ist ausgewählt aus der Gruppe bestehend aus
xi) aromatischen Resten, wobei die aromatischen Reste optional Substituenten, die ausgewählt sind aus der Gruppe bestehend aus Halogen-Resten, Cyano-Resten, Ester-Resten, Keton-Resten, Ether-Resten und Thioether-Resten, tragen,
und xii) linearen, verzweigten und cyclischen aliphatischen C₃- bis C₁₂-Resten.

Optional ist R¹ - bei beiden Möglichkeiten xi) und xii) - ein divalenter Rest, der mit einer Valenz an den Benzolring angebunden ist.

Der aromatische Rest aus der Untergruppe xi) ist beispielsweise und bevorzugt ausgewählt aus Phenyl-Resten (-C₆H₅) und Benzyl-Resten (-CH₂-C₆H₅), wobei Phenyl besonders bevorzugt ist.

Die aromatischen Reste der Untergruppe xi) können dabei Substituenten tragen. Wie oben ausgeführt sind diese ausgewählt aus der Gruppe bestehend aus Halogen-Resten, Cyano-Resten, Ester-Resten, Keton-Resten, Ether-Resten und Thioether-Resten.

Bevorzugt sind die Substituenten dabei ausgewählt aus der Gruppe bestehend aus Ester-Resten, Keton-Resten, Ether-Resten und Thioether-Resten.

Gemäß bevorzugter Ausführungsformen ist der aromatische Rest dabei an den beiden C-Atomen, die dem C₁-Atom, also dem Kohlenstoffatom, welches an das N-Atom gebunden ist, benachbart sind, nicht substituiert. Im Fall eines Benzolringes als Grundgerüst ist somit bevorzugt kein Substituent in ortho-Position zum N-Atom vorhanden.

Gemäß weiterer bevorzugter Ausführungsformen ist der aromatische Rest der Untergruppe xi) nicht substituiert.

Es ist bevorzugt, dass R¹ über ein tertiäres Kohlenstoffatom an das Stickstoffatom (N) gebunden ist. Damit ist das C₁-Atom bevorzugt ein tertiäres Kohlenstoffatom. Unter dem Begriff "tertiäres Kohlenstoffatom" ist im Rahmen der vorliegenden Erfindung ein Kohlenstoffatom zu verstehen, welches an nur ein Wasserstoffatom gebunden ist.

Hiermit ergibt sich eine besonders gute Löslichkeit der erfindungsgemäßen Verbindung in Kautschukmischungen, insbesondere für Fahrzeugreifen und andere technische Gummiartikel, sowie eine optimierte Reaktivität im Zusammenhang mit den für den Alterungsschutz relevanten Mechanismen.

Gemäß vorteilhafter Ausführungsformen ist R¹, insbesondere in den oben genannten Formeln I), II), IIa), ein Phenyl-Rest.

Gemäß einer besonders vorteilhaften Ausführungsform weist die erfindungsgemäße Verbindung die Struktur gemäß Formel III) auf:

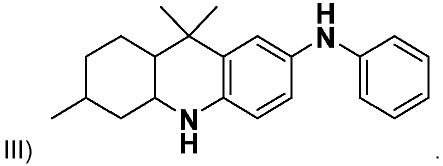

Die Verbindung gemäß Formel III) weist eine besonders gute Löslichkeit in Polymeren auf, insbesondere in Kautschukmischungen für Fahrzeugreifen und andere technische Gummiartikel. Gleichzeitig lässt sich die Verbindung gemäß Formel II) besonders einfach sowie energie- und kostensparend herstellen und zeigt im Vergleich zu 6PPD eine vergleichbare Reaktivität gegenüber Sauerstoff, Ozon oder Radikalen und damit eine vergleichbare Alterungsschutzwirkung.

Die Verbindung gemäß Formel III) kann nach IPUAC auch als 6,9,9-Trimethyl-N-phenyl-5,6,7,8,8a,9,10,10a-octahydroacridin-2-amin bezeichnet werden.

Gemäß weiterer vorteilhafter Ausführungsformen ist R¹, insbesondere in den oben genannten Formeln I), II), IIa), ein verzweigter oder cyclischer Alkyl-Rest mit drei bis zwölf Kohlenstoffatomen, bevorzugt drei bis acht Kohlenstoffatomen, ist, wobei R¹ besonders bevorzugt ausgewählt ist aus 1,3-Dimethylbutyl- und Cyclohexyl-Resten, wobei R¹ ganz besonders bevorzugt ein 1,3-Dimethylbutyl-Rest ist.

Hiermit wird eine besonders gute Löslichkeit in Kautschukmischungen für Fahrzeugreifen und andere technische Gummiartikel erzielt.

Gemäß weiterer vorteilhafter Ausführungsformen ist R¹ in Formel I) ein divalenter Rest, der mit einer Valenz an den Benzolring angebunden ist.

Hierdurch kann m naturgemäß nur noch die Werte 0 oder 1 oder 2 annehmen, da eine Valenz am Benzolring durch R¹ belegt ist.

Bevorzugt ist der divalente Rest ein aliphatischer Rest.

Beispielsweise und besonders bevorzugt weist die Verbindung dabei die Struktur gemäß Formel IV) auf: 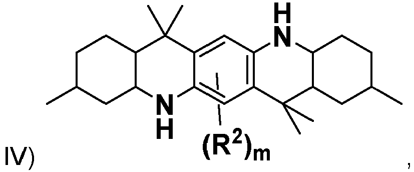 wobei R² wie obenstehend definiert ist, und wobei m den Wert 0 oder 1 oder 2 annimmt.

Bevorzugt nimmt m in Formel IV) den Wert 0 an.

Ein weiterer Gegenstand der vorliegenden Erfindung ist wie oben ausgeführt ein Verfahren zur Herstellung der Verbindung gemäß Formel I), welches wenigstens die folgenden Verfahrensschritte umfasst:
a1) Bereitstellung der Substanz gemäß Formel A1): 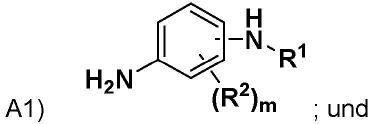
b1) Bereitstellung der Substanz gemäß Formel B1): 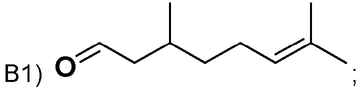 und
c1) Umsetzung der Substanzen gemäß Schritt a1) und b1) in Gegenwart einer ionischen Flüssigkeit, bevorzugt 1-Butyl-3-methylimidazoliumtetrafluoroborat (BMIM BF₄), zu der Substanz gemäß Formel I):

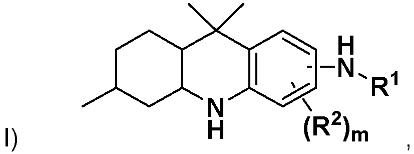

wobei R¹ ausgewählt ist aus der Gruppe bestehend aus xi) aromatischen Resten, wobei die aromatischen Reste optional Substituenten, die ausgewählt sind aus der Gruppe bestehend aus Halogen-Resten, Cyano-Resten, Ester-Resten, Keton-Resten, Ether-Resten und Thioether-Resten, tragen,
und xii) linearen, verzweigten und cyclischen aliphatischen C₃- bis C₁₂-Resten, wobei R¹ optional ein divalenter Rest ist, der mit einer Valenz an den Benzolring angebunden ist; und
wobei R² ausgewählt ist aus der Gruppe bestehend aus linearen, verzweigten und cyclischen aliphatischen C₁- bis C₁₂-Resten, welche optional einen oder mehrere Halogen-Substituenten tragen, Aryl-Resten, welche optional einen oder mehrere Halogen-Substituenten tragen, und Halogen-Resten, wobei Fluor, Brom und Chlor bevorzugt sind, Cyano-Resten, Ester-Resten, Keton-Resten, Ether-Resten und Thioether-Resten; und wobei m den Wert 0 oder 1 oder 2 oder 3 annimmt.

Für die Reste R¹ und R² sowie m gelten sämtliche obigen im Zusammenhang mit der Beschreibung der erfindungsgemäßen Verbindung getroffenen Ausführungen inklusive bevorzugter Ausführungsformen.

Bevorzugt sind die beiden Stickstoffatome der Substanz in der Formel A1) in para-Stellung zueinander angeordnet.

Die Substanz gemäß Formel A1) ist beispielsweise für R¹ gleich Phenyl kommerziell erhältlich.

Die Substanz gemäß Formel B1) ist auch unter dem Trivialnamen Citronellal bekannt. Von Citronellal gibt es wie dem Fachmann bekannt ist, zwei Enantiomere, nämlich (R)-(+)-Citronellal und (S)-(-)-Citronellal, welche auch als Racemat vorliegen können.

Die Umsetzung gemäß Schritt c1) erfolgt in Anlehnung an die Literatur, s. J. S. Yadav et al., Tetrahydron letters 46, (2005), 1039-1044, wobei zudem die unten beschriebenen Verbesserungen identifiziert werden konnten.

Die hergestellte Verbindung gemäß Formel I) fällt beim erfindungsgemäßen Verfahren wie in der Literatur beschrieben als 1:1 Diastereomerengemisch an, beispielsweise, wenn als Substanz gemäß Formel B1) das Enantiomer (R)-(+)-Citronellal verwendet wird.

Die Umsetzung in Schritt c1) findet in Gegenwart einer ionischen Flüssigkeit statt. Dem Fachmann sind ionische Flüssigkeiten bekannt. Hierbei handelt es sich um Salze mit einem vergleichsweise niedrigen Schmelzpunkt.

Die ionische Flüssigkeit dient bevorzugt gleichzeitig als Lösungsmittel und als Katalysator.

Bevorzugt wird eine hydrophile ionische Flüssigkeit verwendet. Der Fachmann kann ionische Flüssigkeiten anhand ihrer Hydrophobie unterscheiden und daher geeignete hydrophile ionische Flüssigkeiten auswählen.

Bevorzugt ist die ionische Flüssigkeit 1-Butyl-3-methylimidazoliumtetrafluoroborat (BMIM BF₄) oder 1-Methyl-3-octylimidazoliumtetrafluoroborat (OMIM BF₄), wobei 1-Butyl-3-methylimidazoliumtetrafluoroborat besonders bevorzugt ist.

Bevorzugt wird eine ionische Flüssigkeit, die bei einer Temperatur von 25 °C flüssig ist, verwendet, also mit einem Schmelzpunkt von weniger als 26 °C.

Bevorzugt findet die Umsetzung in Schritt c1) bei Raumtemperatur statt. Das bedeutet, dass das Reaktionsmedium nicht durch Wärmezufuhr von außen erwärmt werden muss bzw. wird. Hierdurch ist das erfindungsgemäße Verfahren vergleichsweise energiesparend.

Dies wird insbesondere auch durch die gleichzeitige Auswahl einer ionischen Flüssigkeit mit einem entsprechend niedrigen Schmelzpunkt ermöglicht.

Die Umsetzung in Schritt c1) erfolgt bevorzugt über mehrere Stunden. Dabei wird das Reaktionsgemisch bevorzugt über mehrere Stunden gerührt, insbesondere von zwei bis zwölf Stunden, besonders bevorzugt von zwei bis sechs Stunden, insbesondere zwei bis vier Stunden.

Es ist zudem bevorzugt, dass 0,8 bis 0,95, besonders bevorzugt 0,9, Äquivalente, der Substanz gemäß Formel B1) auf ein Äquivalent der Substanz gemäß Formel A1) eingesetzt werden.

Hierdurch reagiert das Citronellal vollständig ab, wodurch eine höhere Ausbeute und Produktreinheit ermöglicht werden. Gleichzeitig erleichtert dies die Wiederverwendbarkeit der ionischen Flüssigkeit.

Bevorzugt erfolgt im Anschluss an Schritt c1) der Verfahrensschritt d1):
d1) Extraktion mit einem organischen Lösungsmittel, bevorzugt mit einer linearen, verzweigten oder cyclischen aliphatischen C₅-C₁₀-Verbindung, besonders bevorzugt mit Cyclohexan.

Bevorzugt erfolgt die Extraktion in Schritt d1) nach Inkontaktbringen des Reaktionsgemisches nach Schritt c1) mit dem Lösungsmittel durch Rühren über einen Zeitraum von einer bis vier Stunden.

Durch die Extraktion wird das Produkt, nämlich die erfindungsgemäße Verbindung gemäß Formel I), aus der ionischen Flüssigkeit entfernt.

Es hat sich dabei herausgestellt, dass die Extraktion mit einer linearen, verzweigten oder cyclischen aliphatischen C₅-C₁₀-Verbindung, besonders bevorzugt mit Cyclohexan besonders vorteilhaft ist. Hierdurch verbleibt überschüssiges Edukt in Form der Verbindung gemäß Formel A1) in der ionischen Flüssigkeit. Damit ist die erfindungsgemäße Verbindung mit einer besonders hohen Produktreinheit herstellbar.

Die ionische Flüssigkeit wird durch die Extraktion somit vom Reaktionsprodukt befreit und enthält nur noch einen Teil der Edukte.

Besonders vorteilhaft ist es dabei, wenn 0,8 bis 0,95, besonders bevorzugt 0,9, Äquivalente der Substanz gemäß Formel B1) auf ein Äquivalent der Substanz gemäß Formel A1) eingesetzt werden, und zudem im Anschluss an Schritt c1) eine Extraktion mit einer linearen, verzweigten oder cyclischen aliphatischen C₅-C₁₀-Verbindung, besonders Cyclohexan, erfolgt.

Hierdurch kann die ionische Flüssigkeit ohne weitere Aufreinigung und somit auf besonders einfache und energiesparende Weise für eine weitere Reaktion gemäß der Schritte a1) bis c1) bereitgestellt und wiederverwendet werden.

Bevorzugt erfolgt zudem in Anschluss an d1) eine Aufreinigung der Lösungsmittelphase, bevorzugt Cyclohexanphase, insbesondere durch Waschen, beispielsweise und bevorzugt mit einer gesättigten Kochsalz-Lösung, und anschließendes Trocknen, beispielsweise und bevorzugt über Natriumsulfat.

Das Lösungsmittel wird bevorzugt im Anschluss auf bekannte Weise, insbesondere im Vakuum, entfernt.

Das erfindungsgemäße Verfahren ermöglicht somit die vergleichsweise einfache sowie energie- und kostensparende Herstellung der erfindungsgemäßen Verbindung gemäß Formel I).

Um die Verbindung gemäß Formel I) mit R¹ beispielsweise gleich 1,3-Dimethylbutyl zu erhalten, kann das Verfahren auch analog durchgeführt werden, wie in den Schemata XII) und XIII) gezeigt: 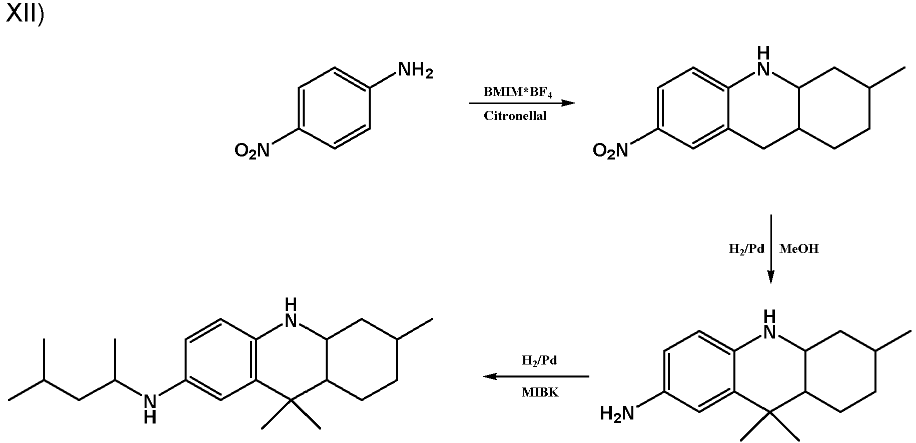 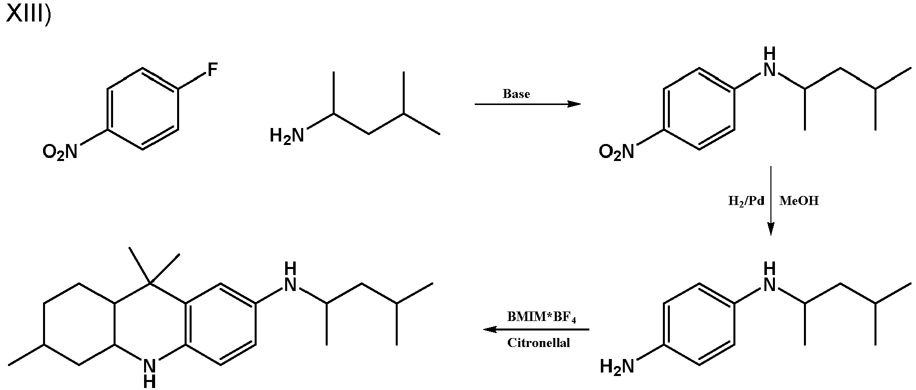 wobei wie dem Fachmann bekannt MeOH für Methanol, H₂ für Wasserstoff, Pd für Palladium und MIBK für Methylisobutylketon stehen.

BMIM*BF₄ steht wie oben ausgeführt für 1-Butyl-3-methylimidazolium-tetrafluoroborat und in den beiden Schemata XII) und XIII) stellvertretend für ionische Flüssigkeiten.

Dabei gelten für die jeweiligen Verfahrensschritte, bei denen eine Umsetzung mit Citronellal in Gegenwart einer ionischen Flüssigkeit erfolgt sämtliche obigen Ausführungen.

Die gezeigten analogen Verfahrensschritte sind insbesondere dann bevorzugt, wenn die Ausgangssubstanz A1) mit dem jeweiligen Rest R¹ nicht kommerziell erhältlich ist.

Ein weiterer Gegenstand der Erfindung ist wie oben ausgeführt eine Kautschukmischung.

Die erfindungsgemäße Kautschukmischung enthält die Verbindung gemäß Formel I), beispielsweise und bevorzugt die Verbindung gemäße Formel III). Bei der erfindungsgemäßen Kautschukmischung kann es sich prinzipiell um jede Kautschukmischung handeln, insbesondere in der die neuartige erfindungsgemäße Verbindung gemäß Formel I) als Alterungsschutzmittel und/oder Ozonschutzmittel bei geringerer Toxizität wirkt.

Die erfindungsgemäße Kautschukmischung enthält wenigstens einen Kautschuk.

Bevorzugt enthält die erfindungsgemäße Kautschukmischung 0,1 bis 10 phr, besonders bevorzugt 0,1 bis 7 phr, ganz besonders bevorzugt 1 bis 6 phr, wiederum bevorzugt 1 bis 3 phr, der Verbindung gemäß Formel I), beispielsweise und bevorzugt die Verbindung gemäße Formel III).

Die in dieser Schrift verwendete Angabe phr (parts per hundred parts of rubber by weight) ist die in der Kautschukindustrie übliche Mengenangabe für Mischungsrezepturen. Die Dosierung der Gewichtsteile der einzelnen Substanzen wird in dieser Schrift auf 100 Gewichtsteile der gesamten Masse aller in der Mischung vorhandenen hochmolekularen (M_{w} größer als 20.000 g/mol) Kautschuke bezogen.

Gemäß vorteilhafter Ausführungsformen der Erfindung enthält die erfindungsgemäße Kautschukmischung wenigstens einen Dienkautschuk.

Die Kautschukmischung kann somit einen Dienkautschuk oder ein Gemisch von zwei oder mehreren verschiedenen Dienkautschuken enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Der Dienkautschuk ist dabei bevorzugt ausgewählt aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Halobutyl-Kautschuk, Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, Silikon-Kautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Insbesondere Nitrilkautschuk, hydrierter Acrylnitrilbutadienkautschuk, Chloroprenkautschuk, Butylkautschuk, Halobutylkautschuk und/oder Ethylen-Propylen-Dien-Kautschuk kommen bei der Herstellung von technischen Gummiartikeln, wie Gurte, Riemen und Schläuche, und/oder Schuhsohlen zum Einsatz. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Bei dem natürlichen und/oder synthetischen Polyisopren sämtlicher Ausführungsformen kann es sich sowohl um cis-1,4-Polyisopren als auch um 3,4-Polyisopren handeln. Bevorzugt ist allerdings die Verwendung von cis-1,4-Polyisoprenen mit einem cis-1,4 Anteil > 90 Gew.-%. Zum einen kann solch ein Polyisopren durch stereospezifische Polymerisation in Lösung mit Ziegler-Natta-Katalysatoren oder unter Verwendung von fein verteilten Lithiumalkylen erhalten werden. Zum anderen handelt es sich bei Naturkautschuk (NR) um ein solches cis-1,4 Polyisopren, bei welchem der cis-1,4-Anteil im Naturkautschuk größer 99 Gew.-% ist.

Ferner ist auch ein Gemisch eines oder mehrerer natürlicher Polyisoprene mit einem oder mehreren synthetischen Polyisopren(en) denkbar.

Im Rahmen der vorliegenden Erfindung ist unter dem Begriff "Naturkautschuk" natürlich vorkommender Kautschuk zu verstehen, der von Hevea Gummibäumen und "Nicht-Hevea" Quellen gewonnen werden kann. Nicht-Hevea Quellen sind beispielsweise Guayule Sträucher und Löwenzahn wie beispielsweise TKS (Taraxacum kok-saghyz; Russischer Löwenzahn).

Falls in der erfindungsgemäßen Kautschukmischung Butadien-Kautschuk (= BR, Polybutadien) enthalten ist, kann es sich um alle dem Fachmann bekannten Typen handeln. Darunter fallen u.a. die sogenannten high-cis- und low-cis-Typen, wobei Polybutadien mit einem cis-Anteil größer oder gleich 90 Gew.-% als high-cis-Typ und Polybutadien mit einem cis-Anteil kleiner als 90 Gew.-% als low-cis-Typ bezeichnet wird. Ein low-cis-Polybutadien ist z.B. Li-BR (Lithium-katalysierter Butadien-Kautschuk) mit einem cis-Anteil von 20 bis 50 Gew.-%. Mit einem high-cis BR werden besonders gute Eigenschaften sowie eine niedrige Hysterese der Kautschukmischung erzielt.

Das oder die eingesetzte(n) Polybutadiene kann/können mit Modifizierungen und Funktionalisierungen endgruppenmodifiziert und/oder entlang der Polymerketten funktionalisiert sein. Bei der Modifizierung kann es sich um solche mit Hydroxy-Gruppen und/oder Ethoxy-Gruppen und/oder Epoxy-Gruppen und/oder Siloxan-Gruppen und/oder Amino-Gruppen und/oder Aminosiloxan und/oder Carboxy-Gruppen und/oder Phthalocyanin-Gruppen und/oder Silan-Sulfid-Gruppen handeln. Es kommen aber auch weitere, dem Fachmann bekannte, Modifizierungen, auch als Funktionalisierungen bezeichnet, in Frage. Bestandteil solcher Funktionalisierungen können Metallatome sein.

Für den Fall, dass wenigstens ein Styrol-Butadien-Kautschuk (Styrol-Butadien-Copolymer) in der Kautschukmischung enthalten ist, kann es sich sowohl um lösungspolymerisierten Styrol-Butadien-Kautschuk (SSBR) als auch um emulsionspolymerisierten Styrol-Butadien-Kautschuk (ESBR) handeln, wobei auch ein Gemisch aus wenigstens einem SSBR und wenigstens einem ESBR eingesetzt werden kann. Die Begriffe "Styrol-Butadien-Kautschuk" und "Styrol-Butadien-Copolymer" werden im Rahmen der vorliegenden Erfindung synonym verwendet.

Das eingesetzte Styrol-Butadien-Copolymer kann mit den oben beim Polybutadien genannten Modifizierungen und Funktionalisierungen endgruppenmodifiziert und/oder entlang der Polymerketten funktionalisiert sein.

Vorzugsweise ist der wenigstens eine Dienkautschuk ausgewählt aus der Gruppe bestehend aus natürlichem Polyisopren (NR, Naturkautschuk), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

Gemäß einer besonders bevorzugten Ausführungsform der Erfindung ist der wenigstens eine Dienkautschuk ausgewählt aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer besonders vorteilhaften Ausführungsform der Erfindung enthält die Kautschukmischung wenigstens ein natürliches Polyisopren (NR) und zwar bevorzugt in Mengen von 50 bis 100 phr, und gemäß einer besonders vorteilhaften Ausführungsform der Erfindung 80 bis 100 phr, ganz besonders bevorzugt 95 bis 100 phr, wiederum bevorzugt 100 phr. Eine derartige Kautschukmischung zeigt insbesondere optimierte Reißeigenschaften und Abriebeigenschaften bei einer guten Verarbeitbarkeit und Reversionsstabilität.

Für den Fall, dass die Kautschukmischung weniger als 100 phr NR enthält, enthält sie bevorzugt als weiteren Kautschuk wenigstens einen Dienkautschuk, der ausgewählt ist aus der Gruppe bestehend aus synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer weiteren besonders vorteilhaften Ausführungsform der Erfindung enthält die Kautschukmischung wenigstens ein natürliches Polyisopren (NR) und zwar bevorzugt in Mengen von 5 bis 55 phr, und gemäß einer besonders vorteilhaften Ausführungsform der Erfindung 5 bis 25 phr, ganz besonders bevorzugt 5 bis 20 phr. Eine derartige Kautschukmischung zeigt insbesondere eine gute Verarbeitbarkeit und Reversionsstabilität sowie optimierte Reißeigenschaften und ein optimales Rollwiderstandsverhalten.

Gemäß einer weiteren besonders vorteilhaften Ausführungsform der Erfindung enthält die Kautschukmischung wenigstens ein Polybutadien (BR, Butadienkautschuk) und zwar bevorzugt in Mengen von 10 bis 80 phr, besonders bevorzugt 10 bis 50 phr, und gemäß einer besonders vorteilhaften Ausführungsform der Erfindung 15 bis 40 phr. Hiermit werden besonders gute Reiß- und Abriebeigenschaften der erfindungsgemäßen Kautschukmischung und ein optimales Bremsverhalten erzielt.

Gemäß einer weiteren besonders vorteilhaften Ausführungsform der Erfindung enthält die Kautschukmischung wenigstens einen lösungspolymerisierten Styrol-Butadien-Kautschuk (SSBR) und zwar bevorzugt in Mengen von 10 bis 80 phr, besonders bevorzugt 30 bis 80 phr, und gemäß einer besonders vorteilhaften Ausführungsform der Erfindung 50 bis 70 phr. Hiermit werden besonders gute Rollwiderstandseigenschaften der erfindungsgemäßen Kautschukmischung erzielt. Gemäß besonders vorteilhaften Ausführungsformen der Erfindung wird SSBR in Kombination mit wenigstens einem weiteren Kautschuk eingesetzt, um ein optimales und ausbalanciertes Eigenschaftsprofil zu erzielen.

Bevorzugt enthält die Kautschukmischung wenigstens einen Füllstoff, bevorzugt in Mengen von 30 bis 500 phr, besonders bevorzugt 50 bis 400 phr, wiederum bevorzugt 80 bis 300 phr.

Gemäß vorteilhafter Ausführungsformen der Erfindung ist der Füllstoff ein verstärkender Füllstoff, der bevorzugt ausgewählt ist aus der Gruppe bestehend aus Rußen und Siliciumdioxid.

Als Ruße kommen alle dem Fachmann bekannten Rußtypen in Frage. Bevorzugt ist der Ruß ausgewählt aus Industrierußen und Pyrolyse-Rußen, wobei Industrieruße weiter bevorzugt sind.

Bevorzugt hat der Ruß eine Jodzahl, gemäß ASTM D 1510, die auch als Jodadsorptionszahl bezeichnet wird, zwischen 30 und 250 g/kg, bevorzugt 30 bis 180 g/kg, besonders bevorzugt 40 bis 180 g/kg, und ganz besonders bevorzugt 40 bis 130 g/kg, und eine DBP-Zahl gemäß ASTM D 2414 von 30 bis 200 ml/100 g, bevorzugt 70 bis 200 ml/100g, besonders bevorzugt 90 bis 200 ml/100g.

Die DBP-Zahl gemäß ASTM D 2414 bestimmt das spezifische Absorptionsvolumen eines Rußes oder eines hellen Füllstoffes mittels Dibutylphthalat.

Die Verwendung eines solchen Rußtyps in der Kautschukmischung, insbesondere für Fahrzeugreifen, gewährleistet einen bestmöglichen Kompromiss aus Abriebwiderstand und Wärmeaufbau, der wiederum den ökologisch relevanten Rollwiderstand beeinflusst.

Besonders geeignet und bevorzugt ist dabei ein Ruß mit einer Jodadsorptionszahl zwischen 80 und 110 g/kg und einer DBP-Zahl von 100 bis 130 ml/100g, wie insbesondere Rußes des Types N339.

Das Siliciumdioxid ist bevorzugt amorphes Siliciumdioxid, beispielsweise gefällte Kieselsäure, die auch als gefälltes Siliciumdioxid bezeichnet wird. Alternativ kann aber beispielsweise auch pyrogenes Siliciumdioxid eingesetzt werden. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 400 m²/g, bevorzugt von 35 bis 350 m²/g, besonders bevorzugt von 85 bis 320 m²/g und ganz besonders bevorzugt von 120 bis 235 m²/g, und eine CTAB-Oberfläche (gemäß ASTM D 3765) von 30 bis 400 m²/g, bevorzugt von 30 bis 330 m²/g, besonders bevorzugt von 80 bis 300 m²/g und ganz besonders bevorzugt von 115 bis 200 m²/g, aufweist. Derartige Kieselsäuren führen z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Als Kieselsäuren können somit z. B. sowohl jene des Typs Ultrasil^{®} VN3 (Handelsname) der Firma Evonik als auch hoch dispergierbare Kieselsäuren, so genannte HD-Kieselsäuren (z. B. Zeosil^{®} 1165 MP der Firma Solvay), zum Einsatz kommen.

Gemäß besonders vorteilhafter Ausführungsformen der Erfindung enthält die Kautschukmischung wenigstens eine Kieselsäure als Füllstoff, bevorzugt in Mengen von 30 bis 500 phr, besonders bevorzugt 50 bis 400 phr, wiederum bevorzugt 80 bis 300 phr.

In diesen Mengen ist Kieselsäure insbesondere als alleiniger oder als Hauptfüllstoff (mehr als 50 Gew.-% bezogen auf die Gesamtfüllstoffmenge) enthalten.

Gemäß weiterer vorteilhafter Ausführungsformen der Erfindung enthält die Kautschukmischung wenigstens eine Kieselsäure als weiteren Füllstoff, und zwar bevorzugt in Mengen von 5 bis 100 phr, besonders bevorzugt 5 bis 80 phr, wiederum bevorzugt 10 bis 60 phr.

In diesen Mengen ist Kieselsäure insbesondere als weiterer Füllstoff zusätzlich zu einem anderen Hauptfüllstoff, wie insbesondere einem Ruß, enthalten.

Die Begriffe "Kieselsäure" und "Silika" werden im Rahmen der vorliegenden Erfindung synonym verwendet.

Gemäß besonders vorteilhafter Ausführungsformen der Erfindung enthält die erfindungsgemäße Kautschukmischung 0,1 bis 60 phr, bevorzugt 3 bis 40 phr, besonders bevorzugt 5 bis 30 phr, ganz besonders bevorzugt 5 bis 15 phr, wenigstens eines Rußes. In diesen Mengen ist Ruß insbesondere als weiterer Füllstoff zusätzlich zu einem Hauptfüllstoff, wie insbesondere Kieselsäure, enthalten.

Gemäß weiterer vorteilhafter Ausführungsformen der Erfindung enthält die erfindungsgemäße Kautschukmischung 30 bis 300 phr, bevorzugt 30 bis 200 phr, besonders bevorzugt 40 bis 100 phr wenigstens eines Rußes. In diesen Mengen ist Ruß als alleiniger oder als Hauptfüllstoff und dabei ggf. in Kombination mit Kieselsäure in den oben genannten geringeren Mengen enthalten.

Gemäß einer besonders vorteilhaften Ausführungsform der Erfindung enthält die Kautschukmischung 5 bis 60 phr, besonders bevorzugt 5 bis 40 phr, wenigstens eines Rußes und 50 bis 300 phr, bevorzugt 80 bis 200 phr wenigstens einer Kieselsäure.

Die Kautschukmischung kann zudem weitere Füllstoffe enthalten, die verstärkend wirken oder nicht verstärkend wirken.

Zu den weiteren (nicht verstärkenden) Füllstoffen zählen im Rahmen der vorliegenden Erfindung Alumosilicate, Kaolin, Kreide, Stärke, Magnesiumoxid, Titandioxid oder Kautschukgele sowie Fasern (wie zum Beispiel Aramidfasern, Glasfasern, Carbonfasern, Cellulosefasern).

Weitere ggf. verstärkende Füllstoffe sind z.B. Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und Carbonyl-Gruppen), Graphit und Graphene und sogenannte "carbon-silica dual-phase filler".

Zinkoxid gehört im Rahmen der vorliegenden Erfindung nicht zu den Füllstoffen.

Des Weiteren kann die Kautschukmischung übliche Zusatzstoffe in üblichen Gewichtsteilen enthalten, die bei deren Herstellung bevorzugt in wenigstens einer Grundmischstufe zugegeben werden. Zu diesen Zusatzstoffen zählen
a) im Stand der Technik bekannte Alterungsschutzmittel,
   wie z. B. p-Phenylendiamine, wie
   N-Phenyl-N'-(1,3-dimethylbutyl)-p-phenylendiamin (6PPD),
   N,N`-Diphenyl-p-phenylendiamin (DPPD),
   N-(1-phenylethyl)-N'-phenyl-p-phenylendiamin (SPPD),
   N,N'-Ditolyl-p-phenylendiamin (DTPD),
   N-(1,4-dimethylpentyl)-N'-phenyl-p-phenylendiamin (7PPD),
   N-Isopropyl-N'-phenyl-p-phenylendiamin (IPPD),
   oder Dihydrochinoline, wie 2,2,4-Trimethyl-1,2-dihydrochinolin (TMQ),
b) Aktivatoren, wie z. B. Zinkoxid und Fettsäuren (z. B. Stearinsäure) und/oder sonstige Aktivatoren, wie Zinkkomplexe wie z.B. Zinkethylhexanoat,
c) Aktivatoren und/oder Agenzien für die Anbindung von Füllstoffen, insbesondere Ruß oder Kieselsäure, wie beispielsweise S-(3-Aminopropyl)Thioschwefelsäure und/oder deren Metallsalze (Anbindung an Ruß) sowie Silan-Kupplungsagenzien (Anbindung an Siliciumdioxid, insbesondere Kieselsäure),
d) Ozonschutzwachse,
e) Harze, insbesondere Klebharze,
f) Mastikationshilfsmittel, wie z. B. 2,2'-Dibenzamidodiphenyldisulfid (DBD) und
g) Prozesshilfsmittel, wie insbesondere Fettsäureester und Metallseifen, wie z.B. Zinkseifen und/oder Calciumseifen
h) Weichmacher, wie insbesondere wie aromatische, naphthenische oder paraffinische Mineralölweichmacher, wie z.B. MES (mild extraction solvate) oder RAE (Residual Aromatic Extract) oder TDAE (treated distillate aromatic extract), oder Rubber-to-Liquid-Öle (RTL) oder Biomass-to-Liquid-Öle (BTL) bevorzugt mit einem Gehalt an polycyclischen Aromaten von weniger als 3 Gew.-% gemäß Methode IP 346 oder Triglyceride, wie z. B. Rapsöl, oder Faktisse oder Kohlenwasserstoffharze oder Flüssig-Polymere, deren mittleres Molekulargewicht (Bestimmung per GPC = gel permeation chromatography, in Anlehnung an BS ISO 11344:2004) zwischen 500 und 20000 g/mol liegt.

Bei der Verwendung von Mineralöl ist dieses bevorzugt ausgewählt aus der Gruppe, bestehend aus DAE (Destillated Aromatic Extracts), RAE (Residual Aromatic Extract), TDAE (Treated Destillated Aromatic Extracts), MES (Mild Extracted Solvents) und naphthenischen Ölen.

Gemäß besonders vorteilhafter Ausführungsformen enthält die erfindungsgemäße Kautschukmischung neben der erfindungsgemäßen Verbindung gemäß Formel I) keine Alterungsschutzmittel aus der Gruppe der p-Phenylendiamine, insbesondere solche unter obiger Auflistung a). Insbesondere enthält die erfindungsgemäße Kautschukmischung gemäß einer besonders bevorzugten Ausführungsform 0 bis 0,1 phr, insbesondere 0 phr, an weiteren Alterungsschutzmitteln auf Basis von p-Phenylendiaminen, die ausgewählt sind aus der Gruppe enthaltend, bevorzugt bestehend aus, N-Phenyl-N'-(1,3-dimethylbutyl)-p-phenylendiamin (6PPD), N-(1-phenylethyl)-N'-phenyl-p-phenylendiamin (SPPD), N,N`-Diphenyl-p-phenylendiamin (DPPD), N,N'-Ditolyl-p-phenylendiamin (DTPD), N-Isopropyl-N'-phenyl-p-phenylendiamin (IPPD), N-(1,4-dimethylpentyl)-N'-phenyl-p-phenylendiamin (7PPD).

Mit den bevorzugt sehr geringen Mengen von 0 bis 0,1 phr bzw. besonders bevorzugt 0 phr an p-Phenylendiaminen, und der erfindungsgemäß enthaltenen Verbindung gemäß Formel I) ist es möglich eine vergleichbare Schutzwirkung bei geringerer Toxizität zu erzielen. Hierbei ersetzt die erfindungsgemäße Verbindung gemäß Formel I) die genannten im Stand der Technik bekannten p-Phenylendiamine.

Gemäß weiterer vorteilhafter Ausführungsformen der Erfindung ist noch wenigstens ein weiteres der genannten p-Phenylendiamin-Alterungsschutzmittel enthalten, sodass die erfindungsgemäße Verbindung die im Stand der Technik bekannten p-Phenylendiamine nur teilweise ersetzt. Hierdurch wird der erfindungsgemäße Vorteil auch erzielt, nur nicht in optimalem Ausmaß.

Alterungsschutzmittel auf Basis von Dihydrochinolin, wie TMQ, sind gemäß vorteilhafter Ausführungsformen neben der erfindungsgemäßen Verbindung gemäß Formel I) in der Kautschukmischung enthalten. Die Menge an enthaltenen Dihydrochinolinen, wie insbesondere TMQ, beträgt bevorzugt 0,1 bis 3, insbesondere 0,5 bis 1,5 phr.

Ozonschutzwachse (obige Gruppe d) werden separat betrachtet und sind gemäß bevorzugter Ausführungsformen der Erfindung in der Kautschukmischung enthalten, unabhängig davon ob zusätzliche Alterungsschutzmittel a) enthalten sind.

Bei den Silan-Kupplungsagenzien kann es sich um alle dem Fachmann bekannten Typen handeln.

Ferner können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen des Siliciumdioxids, insbesondere der Kieselsäure, oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung).

Aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln: -SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3'-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT in verschiedenen Varianten von der Firma Momentive, USA, wie insbesondere 3-Octanoylthio-1-propyltriethoxysilan, oder solche, die unter dem Namen VP Si 363^{®} von der Firma Evonik Industries vertrieben werden.

Der Mengenanteil der Gesamtmenge an weiteren Zusatzstoffen beträgt bevorzugt 3 bis 150 phr, besonders bevorzugt 3 bis 100 phr und ganz besonders bevorzugt 5 bis 80 phr.

Im Gesamtmengenanteil der weiteren Zusatzstoffe kann Zinkoxid (ZnO) in den oben genannten Mengen enthalten sein.

Hierbei kann es sich um alle dem Fachmann bekannten Typen an Zinkoxid handeln, wie z.B. ZnO-Granulat oder -Pulver. Das herkömmlicherweise verwendete Zinkoxid weist in der Regel eine BET-Oberfläche von weniger als 10 m²/g auf. Es kann aber auch ein Zinkoxid mit einer BET-Oberfläche von 10 bis 100 m²/g, wie z.B. so genannte "nano-Zinkoxide", verwendet werden.

Die erfindungsgemäße Kautschukmischung wird bevorzugt vulkanisiert verwendet, insbesondere in Fahrzeugreifen oder anderen vulkanisierten technischen Gummiartikeln.

Die Begriffe "vulkanisiert" und "vernetzt" werden im Rahmen der vorliegenden Erfindung synonym verwendet.

Die Vulkanisation der erfindungsgemäßen Kautschukmischung wird bevorzugt in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern, Mercaptobeschleunigern, Sulfenamidbeschleunigern, Thiocarbamatbeschleunigern, Thiurambeschleunigern, Thiophosphatbeschleunigern, Thioharnstoffbeschleunigern, Xanthogenat-Beschleunigern und Guanidin-Beschleunigern.

Bevorzugt ist die Verwendung eines Sulfenamidbeschleunigers, der ausgewählt ist aus der Gruppe bestehend aus N-Cyclohexyl-2-benzothiazolsufenamid (CBS), N,N-Dicyclohexylbenzothiazol-2-sulfenamid (DCBS), Benzothiazyl-2-sulfenmorpholid (MBS), N-tert-Butyl-2-benzothiazylsulfenamid (TBBS) und Guanidin-Beschleunigern wie Diphenylguanidin (DPG).

Als schwefelspendende Substanz können dabei alle dem Fachmann bekannten schwefelspendenden Substanzen verwendet werden.

Außerdem können in der Kautschukmischung Vulkanisationsverzögerer vorhanden sein.

Die Herstellung der Kautschukmischung erfolgt bevorzugt ansonsten nach dem in der Kautschukindustrie üblichen Verfahren, bei dem zunächst in ein oder mehreren Mischstufen eine Grundmischung mit allen Bestandteilen außer dem Vulkanisationssystem (z. B. Schwefel und vulkanisationsbeeinflussende Substanzen) hergestellt wird. Durch Zugabe des Vulkanisationssystems in einer letzten Mischstufe wird die Fertigmischung erzeugt.

Die Fertigmischung wird z.B. durch einen Extrusionsvorgang oder Kalandrieren weiterverarbeitet und in die entsprechende Form gebracht.

Die erfindungsgemäße Kautschukmischung ist besonders für die Verwendung in Fahrzeugreifen, insbesondere Fahrzeugluftreifen geeignet. Hierbei ist die Anwendung in allen Reifenbauteilen prinzipiell denkbar, insbesondere in einem äußeren Bauteil, insbesondere und bevorzugt im Hornprofil, Laufstreifen und/oder der Seitenwand Laufstreifen. Im Falle eines Laufstreifens mit Cap/Base-Konstruktion wird die erfindungsgemäße Kautschukmischung bevorzugt wenigstens in der Cap verwendet.

Zur Verwendung in Fahrzeugreifen wird die Mischung als Fertigmischung vor der Vulkanisation in die entsprechende Form, bevorzugt eines äußeren Bauteils, gebracht und bei der Herstellung des Fahrzeugreifenrohlings wie bekannt aufgebracht.

Die Herstellung der erfindungsgemäßen Kautschukmischung zur Verwendung als sonstige Body-Mischung in Fahrzeugreifen erfolgt wie bereits beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Als Body-Mischung werden hierbei die Kautschukmischungen für die inneren Bauteile eines Reifen bezeichnet, wie im Wesentlichen Squeegee, Innenseele (Innenschicht), Kernprofil, Gürtel, Schulter, Gürtelprofil, Karkasse, Wulstverstärker, Wulstprofil, Hornprofil und Bandage.

Der noch unvulkanisierte Reifenrohling wird anschließend vulkanisiert.

Zur Verwendung der erfindungsgemäßen Kautschukmischung in Riemen und Gurten, insbesondere in Fördergurten, wird die extrudierte noch unvulkanisierte Mischung in die entsprechende Form gebracht und dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

Ein weiterer Gegenstand der vorliegenden Erfindung ist ein Fahrzeugreifen, der die erfindungsgemäße Kautschukmischung enthaltend die erfindungsgemäße Verbindung in wenigstens einem Bauteil aufweist.

Der vulkanisierte Fahrzeugreifen weist wenigstens in einem Bauteil ein Vulkanisat wenigstens einer erfindungsgemäßen Kautschukmischung auf. Dem Fachmann ist bekannt, dass die meisten Substanzen, wie z. B. die enthaltenen Kautschuke entweder bereits nach dem Mischen oder erst nach der Vulkanisation in chemisch veränderter Form vorliegen oder vorliegen können.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Bevorzugt weist der erfindungsgemäße Fahrzeugreifen die erfindungsgemäße Kautschukmischung in wenigstens einem äußeren Bauteil auf, wobei das äußere Bauteil bevorzugt ein Laufstreifen, eine Seitenwand und/oder ein Hornprofil ist.

Der erfindungsgemäße Fahrzeugreifen kann die erfindungsgemäße Kautschukmischung enthaltend die erfindungsgemäße Verbindung gemäß Formel I) somit auch in mehreren Bauteilen in ggf. angepasster Zusammensetzung aufweisen.

Im Folgenden soll die Erfindung anhand von Ausführungsbeispielen näher erläutert werden.

Die Verbindung gemäß Formel III) als eine beispielhafte Ausführungsform der Verbindung gemäß Formel I) wurde auf folgende Weise, wie in Schema X1) dargestellt, hergestellt:

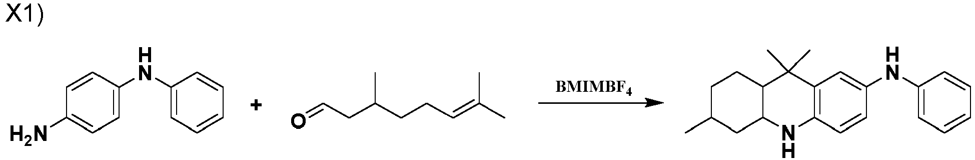

Hierzu wurden 5.00 g *N*-Phenyl-*p*-phenylendiamin (27.1 mmol, 1 Äquivalent (Äq.)), 3.76 g (R)-Citronellal (24.4 mmol, 0.9 Äq.) mit 25 mL BMIMBF₄ vermischt und über Nacht bei Raumtemperatur (RT) rühren gelassen. Anschließend wurden 100 mL Cyclohexan zugegeben und nochmals eine Stunde rühren gelassen. Die Cyclohexanphase wurde abgetrennt, mit gesättigter Natriumchlorid-Lösung gewaschen und über Natriumsulfat (Na₂SO₄) getrocknet. Nachdem die organischen Salze per Filtration entfernt wurden, wurde das Lösungsmittel im Vakuum entfernt. Brauner bis schwarzes viskoses Öl; Ausbeute 7.74 g (99 % d. Th.).

Das Produkt wurde dabei als 1:1 Diasteromerengemisch erhalten, wie beispielsweise anhand der untenstehenden ¹H-NMR sowie ¹³C-NMR-Daten erkennbar.

¹H-NMR (*engl.* "nuclear magnetic resonance") (500 MHz, DMSO-*d*₆) δ = 7.43 (s, 1H), 7.39 (s, 1H), 7.12 - 7.03 (m, 4H), 6.93 (d, *J* = 2.4 Hz, 1H), 6.83 (d, *J* = 2.4 Hz, 1H), 6.76 (ddd, *J* = 8.3, 7.1, 1.3 Hz, 4H), 6.70 (dd, *J* = 8.4, 2.3 Hz, 2H), 6.61 - 6.53 (m, 2H), 6.43 (dd, *J* = 10.5, 8.4 Hz, 2H), 5.33 (s, 2H), 3.70 (q, *J* = 3.1 Hz, 1H), 2.96 (td, *J =* 10.2, 4.1 Hz, 1H), 1.95 (dd, *J*= 12.3, 2.1 Hz, 1H), 1.88 - 1.71 (m, 3H), 1.68 - 1.45 (m, 4H), 1.40 (s, 2H), 1.23 (d, *J =* 8.2 Hz, 6H), 1.19 - 1.11 (m, 6H), 1.03 (s, 3H), 0.99 - 0.83 (m, 9H).

¹³C-NMR (126 MHz, DMSO) δ = 147.6, 147.5, 139.9, 131.3, 131.0, 130.8, 129.4, 129.4, 128.0, 121.0, 121.0, 120.6, 120.4, 117.2, 117.0, 114.3, 113.8, 113.7, 50.4, 47.3, 46.5, 44.3, 43.2, 35.7, 35.3, 35.0, 34.7, 30.8, 27.8, 27.3, 26.8, 26.5, 26.0, 25.5, 24.8, 23.2, 22.7.

ESI-MS (Elektrosprayionisation Massenspektrometrie) [M+H]⁺ = 321.

### Messung der Oxidations-Induktions-Zeit (OIT, engl. "oxidation induction time")

Die Verbindung gemäß Formel III) wurde durch Messung der Oxidations-Induktions-Zeit unter Laborbedingungen auf ihre potenzielle Schutzwirkung als Alterungsschutzmittel untersucht.

Hierzu wurden die Verbindungen gemäß Formel III) sowie 6-PPD jeweils zusammen mit einem Polymer (flüssiges synthetisches Polyisopren (IR), LIR-50, Fa. Kuraray, Gewichtsmittel der Molekulargewichtsverteilung M_{w} = 54.000 g/mol, Glasübergangstemperatur T_{g} = -63°C) bei konstanter Temperatur (180°C) erhitzt bis Oxidation eintrat (Starttemperatur 35 °C, Aufheizen auf 170°C mit einer Heizrate von 20 K/min (Kelvin pro Minute), Aufheizen auf 180°C mit einer Heizrate von 1 K/min; Spülgas: Stickstoff (N₂) mit einem Volumenstrom von 50 mL/min).

Die Probe wurde 5 Minuten bei 180°C isotherm unter N₂-Atmosphäre gehalten und anschließend wurde auf O₂-Atmosphäre (mit einem Volumenstrom von 50 mL/min) umgestellt.

Die Oxidation wurde über einen Peak mittels DSC (dynamische Differenzkalorimetrie; *engl.* "differential scanning calorimetry") ermittelt.

Gemessen wurde die Zeit in Minuten bis zur Oxidation.

Die Ergebnisse sind im Vergleich zu dem bekannten Alterungsschutzmittel 6PPD in Tabelle 1 zusammengefasst.

**Tabelle 1**

| **Substanz** | **Zeit [min] bei 180 °C** |
|---|---|
| 6PPD | 116 ± 10 |
| Formel III) | 93 ± 10 |

Unter Berücksichtigung der Messgenauigkeit von ± (Plusminus) 10 Minuten zeigt sich, dass die Verbindung gemäß Formel III) eine gegenüber 6PPD vergleichbare Schutzwirkung erzielt.

Damit ist die erfindungsgemäße Verbindung gemäß Formel III) als Vertreter der erfindungsgemäßen Verbindung gemäß Formel I) umwelt- und gesundheitsfreundlicher als 6-PPD bzw. weitere Vertreter der Substanzklasse, wie eingangs ausgeführt, und zudem ein vergleichbares Alterungsschutzmittel. Gleichzeitig weist die erfindungsgemäße Verbindung gemäß Formel III) als Vertreter der erfindungsgemäßen Verbindung gemäß Formel I) eine sehr gute Löslichkeit in Kautschukmischungen auf. Hierdurch wird ein Ausblühen vermieden, was wiederum eine verbesserte Schutzwirkung bedeutet.

Mit der Verbindung gemäß Formel I) kann somit eine vergleichbare bzw. im Hinblick auf das verringerte Ausblüh-Verhalten sogar eine verbesserte Schutzwirkung bei den genannten Anwendungsmöglichkeiten erzielt werden.

Ferner wurden zwei weitere erfindungsmäße Verbindungen hergestellt, nämlich 3,14,14-trimethyl-2,3,4,4a,5,12,14,14a-octahydroquinolino[2,3-b]acridin-7(1H)-one (Formel IIa) mit divalentem R¹ und m = 0) und 3,7,7,10,14,14-Hexamethyl-1,2,3,4,4a,5,7,7a,8,9,10,11,11a,12,14,14a-hexadecahydroquinolino[2,3-b]acridin (Formel IV) mit m = 0), nach folgenden Synthesevorschriften:

### Synthese von 3,14,14-trimethyl-2,3,4,4a,5,12,14,14a-octahydroquinolino[2,3-b]acridin-7(1H)-one:

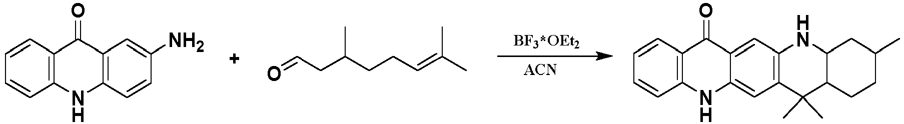

Unter Argon wurden 5.50 g (26.2 mmol, 1 Äq) 2-Aminoacridin-9(10H)on in 200mL trockenem Acetonitril gelöst und 4.25 mL (23.5mmol, 0.9 Äq) Citronellal zugegeben. Anschließend wurden 33 µl (0.26 mmol, 0.1 Äq) Bortrifluoridetherat zugetropft und über Nacht rühren gelassen. Nach Beendigung der Reaktion wurde das Reaktionsgemisch auf Eis gegossen und mit Dichlormetahn extrahiert. Eventuell entstehender Feststoff, welcher Produkt darstellt, wurde per Filtration entfernt. Die Mutterlauge wurde mit gesättigter Kochsalzlösung gewaschen, über Natriumsulfat getrocknet und das Natriumsulfat wurde per Filtration entfernt. Im Anschluss wurde das Lösungsmittel abdestilliert und das Produkt mittels Säulenchromatographie (Cyclohexan/Essigester; 4:1) aufgereinigt. Oranger Feststoff; Ausbeute 6.30 g (70 % d. Th.).

Aufgrund von Diastereomeren werden zwei Signalsätze erhalten, was jedoch nicht die Reinheit der Verbindung und deren Wirkweise beeinträchtigt (Reinheitsprüfung via LC-MS/UV-VIS). Das erfindungsgemäße Molekül wird als Mischung beider Diastereomeren eingesetzt. Im Folgenden sind die H-NMR-Daten der beiden einzelnen Diastereomere angegeben, welche mittels Säulenchromatographie getrennt werden können.

¹H-NMR (500 MHz, DMSO-d6) δ = 11.11 (s, 1 H), 8.06 (dd, J = 8.1, 1.4 Hz, 1H), 7.53 (ddd, *J =* 8.4, 6.9, 1.6 Hz, 1H), 7.35 (dt, *J =* 8.4, 0.8 Hz, 1H), 7.13 (d, *J* = 8.9 Hz, 1H), 7.09 - 7.03 (m, 2H), 5.59 (s, 1H), 3.80 (d, *J* = 3.2 Hz, 1H), 1.87 (dt, J = 13.6, 2.9 Hz, 1H), 1.76 (s, 3H), 1.59 - 1.52 (m, 1H), 1.47 - 1.42 (m, 1H), 1.40 (s, 3H), 1.20 - 1.11 (m, 3H), 0.87 (d, *J* = 6.5 Hz, 3H), 0.81 (dd, *J* = 12.2, 3.0 Hz, 1H), 0.72 (qd, *J* = 12.8, 3.3 Hz, 1H).

¹H-NMR (500 MHz, DMSO-d6) δ = 11.20 (s, 1H), 8.12 (dd, *J =* 8.1, 1.5 Hz, 1H), 7.55 (ddd, *J* = 8.3, 6.8, 1.5 Hz, 1H), 7.40 - 7.35 (m, 1H), 7.18 (d, *J* = 8.9 Hz, 1H), 7.09 (ddd, *J* = 8.1, 6.9, 1.1 Hz, 1H), 7.01 (d, *J* = 8.8 Hz, 1H), 5.56 (d, *J =* 1.4 Hz, 1H), 3.04 (dddt, *J* = 10.6, 8.6, 4.3, 2.1 Hz, 1H), 2.02 (s, 1H), 1.84 (dp, *J* = 12.8, 2.9 Hz, 1H), 1.80 - 1.72 (m, 1H), 1.64 (s, 3H), 1.52 (dddd, *J =* 15.8, 12.6, 6.8, 1.4 Hz, 1H), 1.42 (s, 3H), 1.29 - 1.22 (m, 1H), 1.00 - 0.83 (m, 6H).

ESI-MS [M+H]⁺ = 347.

### Synthese von 3,7,7,10,14, 14-Hexamethyl-1,2,3,4,4a,5,7,7a,8,9, 10, 11, 11a, 12, 14, 14a-hexadeca hydroquinolino[2,3-b]acridin:

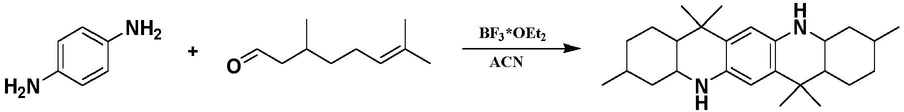

Unter Argon wurden 3 g ( 27.7 mmol, 1.0 Äq) p-Phenylendiamin in 60 mL trockenem Acetonitril gelöst und 10.07 mL (55.5 mmol, 2.0 Äq) Citronellal zugegeben. Anschließend wurden 70 µl (0.5 mmol, 0.02 Äq) Bortrifluoridetherat zugetropft und 5 Stunden rühren gelassen. Nach Beendigung der Reaktion wurde das Reaktionsgemisch auf Eis gegossen und mit Dichlormetahn extrahiert. Die organische Phase wurde mit gesättigter Kochsalzlösung gewaschen, über Natriumsulfat getrocknet und das Natriumsulfat wurde per Filtration entfernt. Im Anschluss wurde das Lösungsmittel entfernt. Braun-roter Feststoff; Ausbeute 10g (95 % d. Th.).

Das erhaltene Gemisch wird ohne weitere Aufreinigung eingesetzt und besteht neben der gewünschten Zielverbindung, die als Diastereomerengemisch vorliegt, aus dem 1:1 sowie 3:1 Reaktionsprodukt von Citronellal und p-Phenylendiamin (s. Tabelle 2). Auch diese Moleküle wirken als Alterungsschutz.

**Tabelle 2**

| Retentionszeit [min] | ESI-MS [M+H]⁺ | Struktur | Ausbeute |
|---|---|---|---|
| 3.87 | 245 | 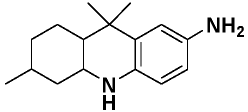 | 4.1 |
| 4.06 | 245 | 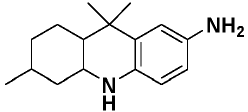 | 3.8 |
| 4.69 | 380 | unbekannt | 4.7 |
| 5.67 | 381 | 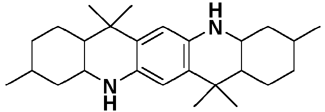 | 36.9 |
| 5.83 | 381 | 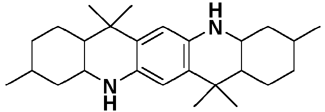 | 30.4 |
| 6.61 | 515 | 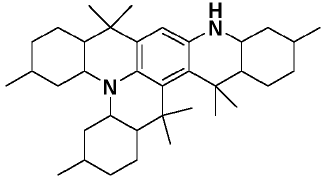 | 6.2 |
| 6.99 | 515 | 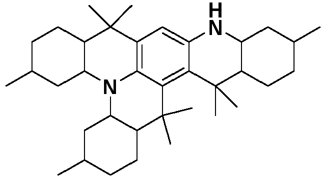 | 8.2 |

Für die Anwendung in einer Kautschukmischung für Fahrzeugreifen wird die erfindungsgemäße Verbindung gemäß Formel I), z. B. die Substanzen gemäß Formel III), 3,14,14-trimethyl-2,3,4,4a,5,12,14,14a-octahydroquinolino[2,3-b]acridin-7(1H)-one oder 3,7,7,10,14,14-Hexamethyl-1,2,3,4,4a,5,7,7a,8,9,10,11,11a,12,14,14a-hexadecahydroquinolino[2,3-b]acridin, beispielsweise anstelle der im Stand der Technik bekannten Alterungsschutzmittel, wie 6PPD, 7PPD oder IPPD usw., auf dem Fachmann bekannte Weise in einer der Mischstufen bei der Herstellung der Kautschukmischung zugegeben.

Die Verbindungen gemäß Formel III) (= Substanz A), 3,14,14-trimethyl-2,3,4,4a,5,12,14,14a-octahydroquinolino[2,3-b]acridin-7(1H)-one (= Substanz B), oder 3,7,7,10,14,14-Hexamethyl-1,2,3,4,4a,5,7,7a,8,9,10,11,11a,12,14,14a-hexadecahydroquinolino[2,3-b]acridin (= Substanz C), werden hierzu beispielsweise in verschiedenen Mengen, wie in Tabelle 3 gezeigt, eingemischt. Die sich ergebenden erfindungsgemäßen Beispiele sind mit E gekennzeichnet.

Als Vergleich dienen Kautschukmischungen enthaltend 6PPD anstelle der vorgenannten Verbindungen als Alterungsschutzmittel bei sonst gleicher Zusammensetzung, wobei zwischen V1 und E1 bis E3 sowie V2 und E4 bis E5 jeweils ein molgleicher Austausch stattfindet. Die Mengen in Tabelle 2 sind in der Einheit phr angegeben. Ferner ist eine Referenz (Ref.) ohne Alterungsschutzmittel angegeben.

Bei sämtlichen Mischungen beträgt die Summe der Mengen an Alterungsschutzmittel (6PPD oder erfindungsgemäße Substanz) und Weichmacheröl MES 10 phr.

**Tabelle 3**

| **Bestandteil** | **V1** | **E1** | **E2** | **E3** | **V2** | **E4** | **E5** | **Ref** |
|---|---|---|---|---|---|---|---|---|
| NR | 100 | 100 | 100 | 100 | 100 | 100 | 100 | 100 |
| Ruß N 339 | 50 | 50 | 50 | 50 | 50 | 50 | 50 | 50 |
| MES | 8 | 7,61 | 7,65 | 8 | 5 | 4,03 | 4,12 | 10 |
| 6PPD | 2 | - | - | - | 5 | - | - | - |
| Substanz A | - | 2,39 | - | - | - | 5,97 | - | - |
| Substanz B | - | - | 2,35 | - | - | - | 5,88 | - |
| Substanz C | - | - | - | 2 | - | - | - | - |
| ZnO | 3 | 3 | 3 | 3 | 3 | 3 | 3 | 3 |
| Stearinsäure | 2 | 2 | 2 | 2 | 2 | 2 | 2 | 2 |
| TBBS | 1,2 | 1,2 | 1,2 | 1,2 | 1,2 | 1,2 | 1,2 | 1,2 |
| Schwefel | 1,2 | 1,2 | 1,2 | 1,2 | 1,2 | 1,2 | 1,2 | 1,2 |

Aus sämtlichen Mischungen der Tabelle 3 wurden Prüfkörper durch 20-minütige Vulkanisation unter Druck bei 160 °C hergestellt und mit diesen Prüfkörpern für die Kautschukindustrie typische Materialeigenschaften mit den im Folgenden angegebenen Testverfahren vor und nach Alterung der Prüfkörper über 28 Tage bei 70 °C in Luft ermittelt:
- Shore-A-Härte bei Raumtemperatur mittels Durometer gemäß DIN ISO 7619-1
- Rückprallelastizität bei Raumtemperatur gemäß ISO 4662
- Spannungswert bei 100 % Dehnung bei Raumtemperatur gemäß DIN 53 504
- Reißdehnung/Bruchdehnung bei Raumtemperatur gemäß DIN 53504

Die ermittelten Messwerte sind in Tabelle 4 aufgelistet.

**Tabelle 4**

| **Eigenschaft** | **Einheit** | **V1** | **E1** | **E2** | **E3** | **V2** | **E4** | **E5** | **Ref** |
|---|---|---|---|---|---|---|---|---|---|
| **Ungealtert** | | | | | | | | | |
| Shorehärte A | ShA | 54,9 | 55,5 | 56,8 | 54,4 | 55,4 | 56,1 | 62,5 | 54,8 |
| Rückprallelast. | % | 46,9 | 44,7 | 46 | 45,5 | 48,2 | 42,6 | 45,9 | 47,8 |
| Spannungswert 100 % | MPa | 1,6 | 1,4 | 1,5 | 1,4 | 1,6 | 1,3 | 1,9 | 1,6 |
| Reißdehnung | % | 612 | 600 | 608 | 650 | 569 | 628 | 570 | 580 |

| **Gealtert: 28 Tage, 70.0 °C in Luft** | | | | | | | | | |
|---|---|---|---|---|---|---|---|---|---|
| Shorehärte A | ShA | 64,3 | 65 | 65,7 | 63,4 | 64,4 | 70,6 | 69,3 | 61,5 |
| Rückprallelast. | % | 51,9 | 49,1 | 49,9 | 49,9 | 47,6 | 44,1 | 49,9 | 49,1 |
| Spannungswert 100 % | MPa | 3 | 2,8 | 2,7 | 2,7 | 2,9 | 2,9 | 3 | 2,7 |
| Reißdehnung | % | 413 | 503 | 495 | 518 | 437 | 512 | 477 | 451 |

Aus der Tabelle 4 wird ersichtlich, dass die Mischungseigenschaften vor Alterung für alle Mischungen auf ähnlichem Niveau liegen. Nach Alterung jedoch zeigt sich, dass die Mischungen mit den erfindungsgemäßen Verbindungen eine bessere Reißdehunung aufweisen als die Mischungen mit 6-PPD. Die erfindungsgemäßen Mischungen zeigen somit eine verbesserte Schutzwirkung vor Alterung im Vergleich zu 6-PPD. Außerdem sind sie umwelt- und gesundheitsfreundlicher als 6-PPD bzw. weitere Vertreter der Substanzklasse und weisen eine sehr gute Löslichkeit in Kautschukmischungen auf. Ein Ausblühen wird vermieden, was wiederum eine verbesserte Schutzwirkung bedeutet.

## Patentansprüche

1. Verbindung gemäß Formel I): 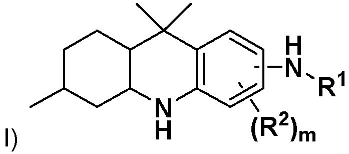
wobei R¹ ausgewählt ist aus der Gruppe bestehend aus xi) aromatischen Resten, wobei die aromatischen Reste optional Substituenten, die ausgewählt sind aus der Gruppe bestehend aus Halogen-Resten, Cyano-Resten, Ester-Resten, Keton-Resten, Ether-Resten und Thioether-Resten, tragen,
und xii) linearen, verzweigten und cyclischen aliphatischen C₃- bis C₁₂-Resten, wobei R¹ optional ein divalenter Rest ist, der mit einer Valenz an den Benzolring angebunden ist; und
wobei R² ausgewählt ist aus der Gruppe bestehend aus linearen, verzweigten und cyclischen aliphatischen C₁- bis C₁₂-Resten, welche optional einen oder mehrere Halogen-Substituenten tragen, Aryl-Resten, welche optional einen oder mehrere Halogen-Substituenten tragen, und Halogen-Resten, wobei Fluor, Brom und Chlor bevorzugt sind, Cyano-Resten, Ester-Resten, Keton-Resten, Ether-Resten und Thioether-Resten; und wobei m den Wert 0 oder 1 oder 2 oder 3 annimmt.

2. Verbindung nach Anspruch 1, **dadurch gekennzeichnet, dass** sie die Struktur gemäß Formel II) aufweist: 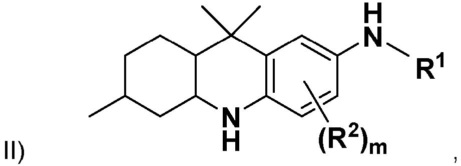 wobei R¹, R² und m wie für Anspruch 1 definiert sind.

3. Verbindung nach Anspruch 2, **dadurch gekennzeichnet, dass** m gleich 0 (null) ist, und die Verbindung damit die Struktur gemäß Formel IIa) aufweist: 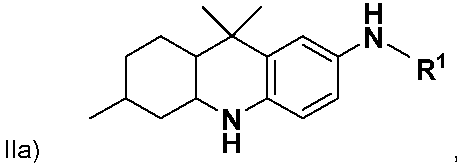 wobei R¹ wie für Anspruch 1 definiert ist.

4. Verbindung nach einem der vorherigen Ansprüche, **dadurch gekennzeichnet, dass** R¹ über ein tertiäres Kohlenstoffatom an das Stickstoffatom (N) gebunden ist.

5. Verbindung nach einem der vorherigen Ansprüche, **dadurch gekennzeichnet, dass** R¹ ein Phenyl-Rest ist.

6. Verbindung nach einem der Ansprüche 1 bis 5, **dadurch gekennzeichnet, dass** sie die Struktur gemäß Formel III) aufweist

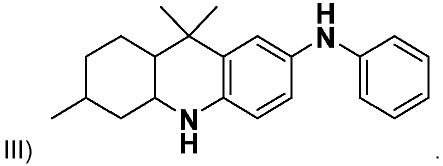

7. Verbindung nach einem der Ansprüche 1 bis 4, **dadurch gekennzeichnet, dass** R¹ ein verzweigter oder cyclischer Alkyl-Rest mit drei bis zwölf Kohlenstoffatomen, bevorzugt drei bis acht Kohlenstoffatomen, ist, wobei R¹ besonders bevorzugt ausgewählt ist aus 1,3-Dimethylbutyl- und Cyclohexyl-Resten, wobei R¹ ganz besonders bevorzugt ein 1,3-Dimethylbutyl-Rest ist.

8. Verbindung nach Anspruch 1 oder 2, **dadurch gekennzeichnet, dass** R¹ ein divalenter Rest ist, der mit einer Valenz an den Benzolring angebunden ist, wobei der divalente Rest bevorzugt aliphatisch ist, und wobei die Verbindung besonders bevorzugt die Struktur gemäß Formel IV) aufweist: 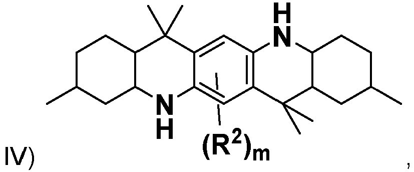 wobei R² wie für Anspruch 1 definiert ist, und wobei m den Wert 0 oder 1 oder 2 annimmt, wobei m bevorzugt den Wert 0 annimmt.

9. Kautschukmischung enthaltend die Verbindung nach einem der Ansprüche 1 bis 8, wobei die Kautschukmischung bevorzugt wenigstens einen Dienkautschuk enthält, der besonders bevorzugt ausgewählt ist aus der Gruppe bestehend aus natürlichem Polyisopren (NR), synthetischem Polyisopren (IR), Butadienkautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

10. Fahrzeugreifen, der die Kautschukmischung nach Anspruch 9 in wenigstens einem Bauteil, bevorzugt in wenigstens einem äußeren Bauteil, aufweist, wobei das äußere Bauteil bevorzugt ein Laufstreifen, eine Seitenwand und/oder ein Hornprofil ist.

11. Verwendung der Verbindung nach einem der Ansprüche 1 bis 8 als Alterungsschutzmittel und/oder Ozonschutzmittel insbesondere in Fahrzeugreifen und/oder anderen technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

12. Verwendung der Verbindung nach einem der Ansprüche 1 bis 8 zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon.

13. Verwendung der Verbindung nach einem der Ansprüche 1 bis 8 in Ölen, Schmierstoffen, wie insbesondere Treib- oder Betriebsstoffen für Motoren.

14. Verfahren zur Herstellung der Verbindung gemäß Formel I), welches die folgenden Verfahrensschritte umfasst:
a1) Bereitstellung der Substanz gemäß Formel A1): 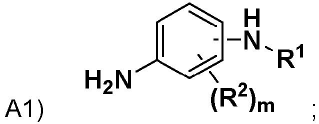 und
b1) Bereitstellung der Substanz gemäß Formel B1): 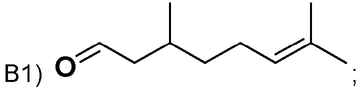 und
c1) Umsetzung der Substanzen gemäß Schritt a1) und b1) in Gegenwart einer ionischen Flüssigkeit, bevorzugt 1-Butyl-3-methylimidazoliumtetrafluoroborat (BMIM BF₄) oder 1-Methyl-3-octylimidazoliumtetrafluoroborat (OMIM BF₄), zu der Substanz gemäß Formel I): 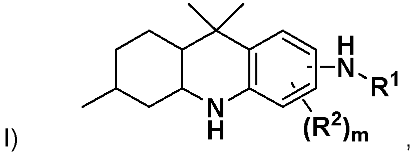
wobei R¹ ausgewählt ist aus der Gruppe bestehend aus xi) aromatischen Resten, wobei die aromatischen Reste optional Substituenten, die ausgewählt sind aus der Gruppe bestehend aus Halogen-Resten, Cyano-Resten, Ester-Resten, Keton-Resten, Ether-Resten und Thioether-Resten, tragen,
und xii) linearen, verzweigten und cyclischen aliphatischen C₃- bis C₁₂-Resten, wobei R¹ optional ein divalenter Rest ist, der mit einer Valenz an den Benzolring angebunden ist; und
wobei R² ausgewählt ist aus der Gruppe bestehend aus linearen, verzweigten und cyclischen aliphatischen C₁- bis C₁₂-Resten, welche optional einen oder mehrere Halogen-Substituenten tragen, Aryl-Resten, welche optional einen oder mehrere Halogen-Substituenten tragen, und Halogen-Resten, wobei Fluor, Brom und Chlor bevorzugt sind, Cyano-Resten, Ester-Resten, Keton-Resten, Ether-Resten und Thioether-Resten; und wobei m den Wert 0 oder 1 oder 2 oder 3 annimmt.

15. Verfahren nach Anspruch 14, **dadurch gekennzeichnet, dass** im Anschluss an Schritt c1) der Verfahrensschritt d1) erfolgt:
d1) Extraktion mit einem organischen Lösungsmittel, bevorzugt mit einer linearen, verzweigten oder cyclischen aliphatischen C₅-C₁₀-Verbindung, besonders bevorzugt mit Cyclohexan.

16. Verfahren nach einem der Ansprüche 14 oder 15, **dadurch gekennzeichnet, dass** 0,8 bis 0,95, bevorzugt 0,9, Äquivalente der Substanz gemäß Formel B1) auf ein Äquivalent der Substanz gemäß Formel A1) eingesetzt werden.

## Claims

1. Compound of formula I): 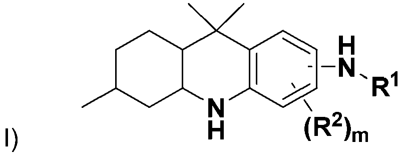
wherein R¹ is selected from the group consisting of xi) aromatic radicals, wherein the aromatic radicals optionally bear substituents selected from the group consisting of halogen radicals, cyano radicals, ester radicals, ketone radicals, ether radicals and thioether radicals,
and xii) linear, branched and cyclic aliphatic C₃- to C₁₂-radicals,
wherein R¹ is optionally a divalent radical bonded to the benzene ring with one valence; and
wherein R² is selected from the group consisting of linear, branched and cyclic aliphatic C₁- to C₁₂-radicals optionally bearing one or more halogen substituents, aryl radicals optionally bearing one or more halogen substituents and halogen radicals, wherein fluorine, bromine and chlorine are preferred, cyano radicals, ester radicals, ketone radicals, ether radicals and thioether radicals; and wherein m assumes the value 0 or 1 or 2 or 3.

2. Compound according to Claim 1, **characterized in that** it has the structure of formula II): 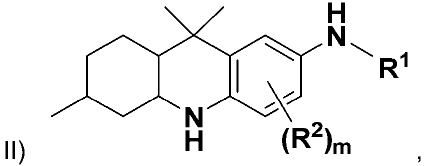 wherein R¹, R² and m are as defined for Claim 1.

3. Compound according to Claim 2, **characterized in that** m is 0 (zero) and the compound thus has the structure of formula IIa): 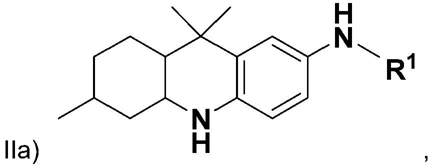 wherein R¹ is as defined for Claim 1.

4. Compound according to any of the preceding claims, **characterized in that** R¹ is bonded to the nitrogen atom (N) via a tertiary carbon atom.

5. Compound according to any of the preceding claims, **characterized in that** R¹ is a phenyl radical.

6. Compound according to any of Claims 1 to 5, **characterized in that** it has the structure of formula III)

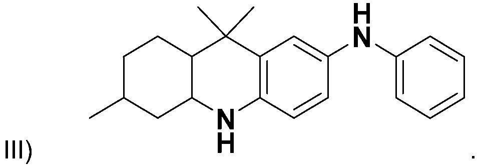

7. Compound according to any of Claims 1 to 4, **characterized in that** R¹ is a branched or cyclic alkyl radical having three to twelve carbon atoms, preferably three to eight carbon atoms, wherein R¹ is particularly preferably selected from 1,3-dimethylbutyl and cyclohexyl radicals, wherein R¹ is very particularly preferably a 1,3-dimethylbutyl radical.

8. Compound according to Claim 1 or 2, **characterized in that** R¹ is a divalent radical which is bonded to the benzene ring with one valence, wherein the divalent radical is preferably aliphatic and wherein the compound particularly preferably has the structure of formula IV): 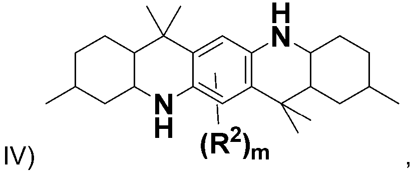 wherein R² is as defined for Claim 1 and wherein m assumes the value 0 or 1 or 2, wherein m preferably assumes the value 0.

9. Rubber mixture containing the compound according to any of Claims 1 to 8, wherein the rubber mixture preferably contains at least one diene rubber which is particularly preferably selected from the group consisting of natural polyisoprene (NR), synthetic polyisoprene (IR), butadiene rubber (BR), solution-polymerized styrene-butadiene rubber (SSBR), emulsion-polymerized styrene-butadiene rubber (ESBR), butyl rubber (IIR) and halobutyl rubber.

10. Vehicle tyre which comprises the rubber mixture according to Claim 9 in at least one component, preferably in at least one external component, wherein the external component is preferably a tread, a sidewall and/or a flange profile.

11. Use of the compound according to any of Claims 1 to 8 as an aging stabilizer and/or antiozonant especially in vehicle tyres and/or other industrial rubber articles, such as especially an air spring, a bellows, a conveyor belt, a strap, a drive belt, a hose, a rubber band, a profile, a seal, a membrane, tactile sensors for medical applications or robotics applications or a shoe sole or parts thereof and/or oils and/or lubricants.

12. Use of the compound according to any of Claims 1 to 8 for producing a rubber article, in particular an air spring, a bellows, a conveyor belt, a strap, a drive belt, a hose, a rubber band, a profile, a seal, a membrane, tactile sensors for medical applications or robotics applications or a shoe sole or parts thereof.

13. Use of the compound according to any of Claims 1 to 8 in oils and lubricants, such as especially fuels or fluids for engines.

14. Process for producing the compound of formula I) which comprises the following process steps:
a1) providing the substance of formula A1): 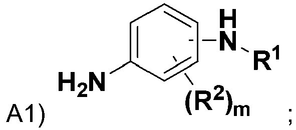 and
b1) providing the substance of formula B1): 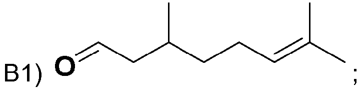 and
c1) reacting the substances of step a1) and b1) in the presence of an ionic liquid, preferably 1-butyl-3-methylimidazolium tetrafluoroborate (BMIM BF₄) or 1-methyl-3-octylimidazolium tetrafluoroborate (OMIM BF₄) to afford the substance of formula I): 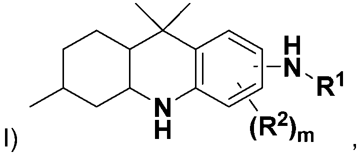
wherein R¹ is selected from the group consisting of xi) aromatic radicals, wherein the aromatic radicals optionally bear substituents selected from the group consisting of halogen radicals, cyano radicals, ester radicals, ketone radicals, ether radicals and thioether radicals,
and xii) linear, branched and cyclic aliphatic C₃- to C₁₂-radicals, wherein R¹ is optionally a divalent radical bonded to the benzene ring with one valence; and
wherein R² is selected from the group consisting of linear, branched and cyclic aliphatic C₁- to C₁₂-radicals optionally bearing one or more halogen substituents, aryl radicals optionally bearing one or more halogen substituents and halogen radicals, wherein fluorine, bromine and chlorine are preferred, cyano radicals, ester radicals, ketone radicals, ether radicals and thioether radicals; and wherein m assumes the value 0 or 1 or 2 or 3.

15. Process according to Claim 14, **characterized in that** step c1) is followed by process step d1):
d1) extracting with an organic solvent, preferably with a linear, branched or cyclic aliphatic C₅-C₁₀ compound, particularly preferably with cyclohexane.

16. Process according to either of Claims 14 or 15, **characterized in that** it employs 0.8 to 0.95, preferably 0.9, equivalents of the substance of formula B1) for one equivalent of the substance of formula A1).

## Revendications

1. Composé selon la formule I) : 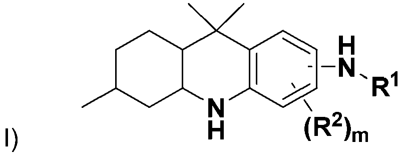
dans laquelle R¹ est choisi dans le groupe constitué par xi) les radicaux aromatiques, les radicaux aromatiques portant éventuellement des substituants qui sont choisis dans le groupe constitué par les radicaux halogène, cyano, ester, cétone, éther et thioéther,
et xii) les radicaux linéaires, ramifiés et cycliques, aliphatiques en C₃-C₁₂,
R¹ représentant éventuellement un radical bivalent, qui est lié à l'aide d'une valence au cycle benzène ; et
dans laquelle R² est choisi dans le groupe constitué par les radicaux linéaires, ramifiés et cycliques, aliphatiques en C₁-C₁₂, qui portent éventuellement un ou plusieurs substituants halogène, les radicaux aryle, qui portent éventuellement un ou plusieurs substituants halogène, et les radicaux halogène, le fluor, le brome et le chlore étant préférés, les radicaux cyano, ester, cétone, éther et thioéther ; et dans laquelle m prend la valeur 0 ou 1 ou 2 ou 3.

2. Composé selon la revendication 1, **caractérisé en ce qu'**il présente la structure selon la formule II) : 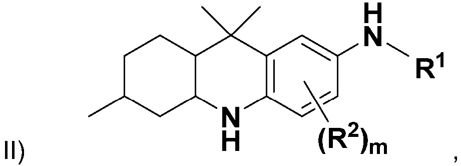 dans laquelle R¹, R² et m sont définis comme pour la revendication 1.

3. Composé selon la revendication 2, **caractérisé en ce que** m vaut 0 (zéro) et le composé présente donc la structure selon la formule IIa) : 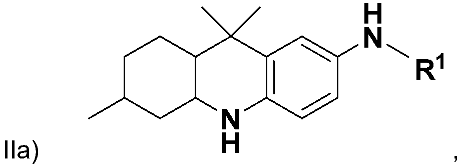 dans laquelle R¹ est défini comme pour la revendication 1.

4. Composé selon l'une des revendications précédentes, **caractérisé en ce que** R¹ est lié à l'atome d'azote (N) par l'intermédiaire d'un atome de carbone tertiaire.

5. Composé selon l'une des revendications précédentes, **caractérisé en ce que** R¹ représente un radical phényle.

6. Composé selon l'une des revendications 1 à 5, **caractérisé en ce qu'**il présente la structure selon la formule III) :

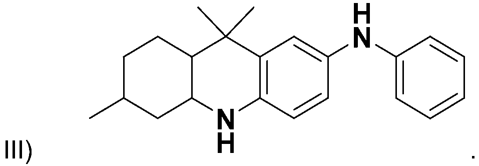

7. Composé selon l'une des revendications 1 à 4, **caractérisé en ce que** R¹ représente un radical alkyle ramifié ou cyclique comprenant trois à douze atomes de carbone, de préférence trois à huit atomes de carbone, R¹ étant choisi de manière particulièrement préférée parmi les radicaux 1,3-diméthylbutyle et cyclohexyle, R¹ représentant de manière tout particulièrement préférée un radical 1,3-diméthylbutyle.

8. Composé selon la revendication 1 ou 2, **caractérisé en ce que** R¹ représente un radical bivalent, qui est lié à l'aide d'une valence au cycle benzène, le radical bivalent étant de préférence aliphatique et le composé présentant de manière particulièrement préférée la structure selon la formule IV) : 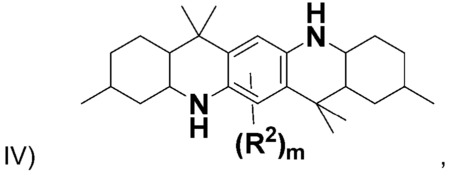 dans laquelle R² est défini comme pour la revendication 1 et m prend la valeur 0 ou 1 ou 2, m prenant de préférence la valeur 0.

9. Mélange de caoutchouc contenant le composé selon l'une des revendications 1 à 8, le mélange de caoutchouc contenant de préférence au moins un caoutchouc diénique, qui est choisi de manière particulièrement préférée dans le groupe constitué par le polyisoprène naturel (NR), le polyisoprène synthétique (IR), le caoutchouc de butadiène (BR), le caoutchouc de styrène-butadiène polymérisé en solution (SSBR), le caoutchouc de styrène-butadiène polymérisé en émulsion (ESBR), le caoutchouc butyle (IIR) et le caoutchouc halogénobutyle.

10. Pneumatique pour véhicule, qui présente le mélange de caoutchouc selon la revendication 9 dans au moins un composant, de préférence dans au moins un composant externe, le composant externe étant de préférence une bande de roulement, un flanc latéral et/ou un profilé de talon.

11. Utilisation du composé selon l'une des revendications 1 à 8 en tant qu'agent de protection contre le vieillissement et/ou agent de protection contre l'ozone, en particulier dans des pneumatiques pour véhicule et/ou d'autres articles techniques en caoutchouc, tels qu'en particulier un ressort pneumatique, un soufflet, une bande transporteuse, une sangle, une courroie, un tuyau, une bande en caoutchouc, un profilé, un joint d'étanchéité, une membrane, des capteurs tactiles pour applications médicales ou applications robotiques, ou une semelle de chaussure ou de parties de celle-ci, et/ou des huiles et/ou des lubrifiants.

12. Utilisation du composé selon l'une des revendications 1 à 8 pour la fabrication d'un article en caoutchouc, en particulier d'un ressort pneumatique, d'un soufflet, d'une bande transporteuse, d'une sangle, d'une courroie, d'un tuyau, d'une bande en caoutchouc, d'un profilé, d'un joint d'étanchéité, d'une membrane, de capteurs tactiles pour applications médicales ou applications robotiques, ou d'une semelle de chaussure ou de parties de celle-ci.

13. Utilisation du composé selon l'une des revendications 1 à 8 dans des huiles, des lubrifiants, tels qu'en particulier des carburants ou fluides opérationnels pour moteurs.

14. Procédé pour la préparation du composé selon la formule I), qui comprend les étapes de procédé suivantes :
a1) mise à disposition de la substance selon la formule A1) : 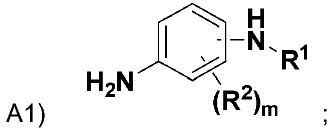 et
b1) mise à disposition de la substance selon la formule B1) : 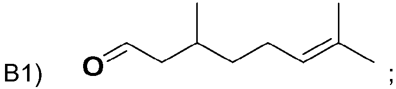 et
c1) transformation des substances selon les étapes a1) et b1) en présence d'un liquide ionique, de préférence le tétrafluoroborate de 1-butyl-3-méthylimidazolium (BMIM BF₄) ou le tétrafluoroborate de 1-méthyl-3-octylimidazolium (OMIM BF₄), en substance selon la formule I) : 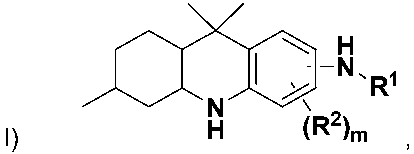
dans laquelle R¹ est choisi dans le groupe constitué par xi) les radicaux aromatiques, les radicaux aromatiques portant éventuellement des substituants qui sont choisis dans le groupe constitué par les radicaux halogène, cyano, ester, cétone, éther et thioéther,
et xii) les radicaux linéaires, ramifiés et cycliques, aliphatiques en C₃-C₁₂, R¹ représentant éventuellement un radical bivalent, qui est lié à l'aide d'une valence au cycle benzène ; et
dans laquelle R² est choisi dans le groupe constitué par les radicaux linéaires, ramifiés et cycliques, aliphatiques en C₁-C₁₂, qui portent éventuellement un ou plusieurs substituants halogène, les radicaux aryle, qui portent éventuellement un ou plusieurs substituants halogène, et les radicaux halogène, le fluor, le brome et le chlore étant préférés, les radicaux cyano, ester, cétone, éther et thioéther ; et dans laquelle m prend la valeur 0 ou 1 ou 2 ou 3.

15. Procédé selon la revendication 14, **caractérisé en ce que** l'étape d1) a lieu consécutivement à l'étape c1) :
d1) extraction à l'aide d'un solvant organique, de préférence d'un composé linéaire, ramifié ou cyclique, aliphatique en C₅-C₁₀, de manière particulièrement préférée à l'aide de cyclohexane.

16. Procédé selon l'une des revendications 14 ou 15, **caractérisé en ce qu'**on utilise 0,8 à 0,95, de préférence 0,9, équivalent de substance selon la formule B1) par rapport à un équivalent de la substance selon la formule A1).
